# DEMANDE DE BREVET EUROPEEN

(11) **EP 4 321 651 A1**
(43) Date de publication de la demande: **14.02.2024**
(21) Numéro de dépôt: 23189899.0
(22) Date de dépôt: 05.08.2023
(51) Int. Cl.: C23G 5/024, C23G 5/032, C11D 1/66, C11D 3/18, C11D 11/00

(54) **COMPOSITION COMPRENANT UN ALCANE ET UN OXO-ESTER**

(30) Priorité: 13.08.2022 FR 2208305
(71) Demandeur: Biosynthis, 91410 Saint Cyr Sous Dourdan (FR)
(72) Inventeur: BERNOUD, Thierry, 91410 SAINT CYR SOUS DOURDAN (FR)
(74) Mandataire: Tripoz, Inès

(57) **Abrégé**

L'invention concerne une composition comprenant au moins un alcane de formule CₙH₂ₙ₊₂ avec n≤12, et au moins un oxo-ester, et un aérosol la comprenant.

L'invention concerne également un procédé pour dégraisser, nettoyer, protéger, dégripper, faire briller et/ou lubrifier une surface d'un objet comprenant l'application de ladite composition.

## Description

La présente demande concerne le domaine technique des compositions dégraissantes, dégrippantes, nettoyantes et/ou lubrifiantes.

L'invention concerne une composition comprenant au moins un alcane de formule CₙH₂ₙ₊₂ avec n≤ 12 et au moins un oxo-ester, et un aérosol la comprenant.

L'invention concerne également un procédé pour dégraisser, nettoyer, protéger, dégripper, faire briller et/ou lubrifier une surface comprenant l'application de la composition.

Le produit WD-40^{®} est un dégraissant multifonction développé en 1953 et largement rependu dans le commerce.

Les ingrédients principaux du WD-40 sont les suivants :
- 50 % d'hydrocarbures aliphatiques,
- Moins de 25 % d'huile de base pétrole,
- Hydrocarbure aliphatique à faible pression de vapeur de 12 à 18 %,
- 2 à 3 % de dioxyde de carbone,
- Moins de 10 % d'ingrédients inertes.

Bien qu'efficace, ce produit est néfaste pour l'environnement en raison de sa composition d'origine pétrochimique. Il est de plus hautement toxique et irritant pour la peau.

Il existe donc un réel besoin de disposer de compositions pour dégraisser, nettoyer, protéger, dégripper, faire briller et/ou lubrifier les surfaces et possédant un meilleur impact à la fois écologique et sur la santé de l'utilisateur.

La demande de brevet CN103510099 divulgue un agent dégraissant respectueux de l'environnement. Ledit dégraissant est constitué majoritairement de carbonate de sodium et de soude et a pour constituants minoritaires notamment des émulsifiants et silicones.

D'autres demandes telles que les demandes CN106011877, CN103614732, CN105220166 décrivent des travaux destinés à fournir un dégraissant respectueux de l'environnement. Aucune de ces demandes ne divulgue l'utilisation d'une composition comprenant au moins un alcane de formule CₙH₂ₙ₊₂ avec n≤12 et au moins un oxo-ester.

Le brevet EP2726649 (ou la demande US201412998) divulgue un procédé de dégraissage de surfaces métalliques comprenant l'application d'une composition comprenant au moins un ester d'acide lévulinique. Cependant, il est démontré dans des essais comparatifs que cette composition présente une mauvaise volatilité, rendant plus complexe sa formulation en aérosol et obligeant également l'utilisateur à rincer ou sécher la surface après l'application. Il a également été démontré que cette composition est inefficace pour traiter, par trempage, les graisses brulées couramment retrouvées notamment sur les pièces mécaniques.

La demande US2007207939A1 a pour objet l'utilisation de mono et di ester d'acide gras de 1,3-propanediol en tant que solvant. Elle ne divulgue pas de composition comprenant au moins un alcane de formule CₙH₂ₙ₊₂ avec 8≤n≤12, et au moins un oxo-ester pour leur utilisation pour dégraisser, nettoyer, protéger, dégripper, faire briller et/ou lubrifier une surface d'un objet.

De manière tout à fait surprenante, un procédé comprenant l'application d'une composition pour dégraisser, nettoyer, protéger, dégripper, faire briller et/ou lubrifier une surface, possédant une bonne volatilité, n'étant ni toxique, ni irritante pour la peau et possédant un bon indice de biodégradabilité et de naturalité a été mis au point par la demanderesse.

Un premier objet selon la présente invention concerne une composition caractérisée en ce qu'elle comprend :
a) au moins un alcane de formule CₙH₂ₙ₊₂ avec n≤ 12, à une teneur de 40% à 95% (v/v) en volume par rapport au volume total de la composition et
b) au moins un oxo-ester de formule I, 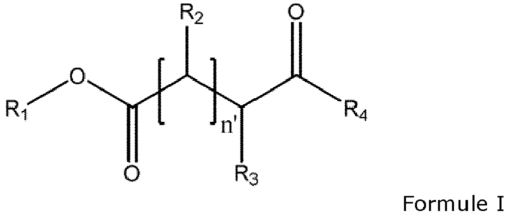 dans laquelle,
   - R₁ est choisi dans le groupe constitué par les alkyles linéaires ou ramifiés comprenant de 1 à 8 atomes de carbone ;
   - R₂, R₃ et R₄ identiques ou différents sont choisis dans le groupe constitué par l'atome d'hydrogène et/ou les alkyles linéaires ou ramifiés comprenant de 1 à 4 atomes de carbone; et
   - n'est un entier naturel de 1 à 4
la teneur en ledit oxo-ester étant de 5% à 60% (v/v) en volume par rapport au volume total de la composition.

Dans un mode de réalisation, la composition comprend :
a) au moins un alcane de formule CₙH₂ₙ₊₂ avec 8≤n≤12, à une teneur de 40% à 95% (v/v) en volume par rapport au volume total de la composition et
b) au moins un oxo-ester de formule I, 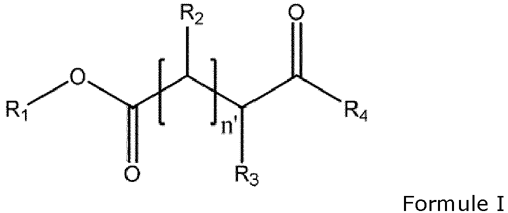 dans laquelle,
   - R₁ est choisi dans le groupe constitué par les alkyles linéaires ou ramifiés comprenant de 1 à 8 atomes de carbone ;
   - R₂, R₃ et R₄ identiques ou différents sont choisis dans le groupe constitué par l'atome d'hydrogène et/ou les alkyles linéaires ou ramifiés comprenant de 1 à 4 atomes de carbone; et
   - n'est un entier naturel de 1 à 4
la teneur en ledit oxo-ester étant de 5% à 60% (v/v) en volume par rapport au volume total de la composition.

Dans un mode de réalisation, la composition selon l'invention ne comprend pas d'ester d'acide gras de 1,3-propanediol.

Dans la présente demande, un « alcane » est un hydrocarbure saturé étant constitué uniquement d'atomes de carbone et d'hydrogène liés entre eux par des liaisons covalentes simples dont la formule générale est CₙH₂ₙ₊₂. Il est appelé « alcane linéaire » lorsque chaque atome de carbone est lié au maximum à deux atomes de carbone et « alcane ramifié » lorsque certains atomes de carbone sont liés à trois atomes, voire quatre atomes de carbones.

Dans la présente demande un « bioalcane » est un alcane biosourcé.

Au sens de la présente invention, on entend par « biosourcé » un composé ou une composition organique dont le carbone organique présent dans le composé ou la composition est à 100% d'origine végétale (¹⁴C) par une analyse au radiocarbone selon l'une des normes suivantes ASTM D6866, EN 16640 ou EN 16785-1.

Dans un mode de réalisation, la composition selon l'invention comprend un mélange d'au moins deux alcanes de formule CₙH₂ₙ₊₂ avec n≤12 différents.

Dans un mode de réalisation, la composition selon l'invention comprend un mélange d'au moins deux alcanes de formule CₙH₂ₙ₊₂ avec 8≤n≤12 différents

Dans un mode de réalisation, au moins un alcane de formule CₙH₂ₙ₊₂ avec n≤12 est un bioalcane.

Dans un mode de réalisation, au moins un alcane de formule CₙH₂ₙ₊₂ avec 8≤n≤12 est un bioalcane.

Dans un mode de réalisation, l'ensemble des alcanes de formule CₙH₂ₙ₊₂ avec n≤12 sont des bioalcanes.

Dans un mode de réalisation, l'ensemble des alcanes de formule CₙH₂ₙ₊₂ avec 8≤n≤12 sont des bioalcanes.

Dans un mode de réalisation, au moins un alcane de formule CₙH₂ₙ₊₂ avec n≤12 a un nombre d'atomes de carbone pair.

Dans un mode de réalisation, au moins un alcane de formule CₙH₂ₙ₊₂ avec 8≤n≤12 a un nombre d'atomes de carbone pair.

Dans un mode de réalisation, l'ensemble des alcanes de formule CₙH₂ₙ₊₂ avec n≤12 ont un nombre d'atomes de carbone pair.

Dans un mode de réalisation, l'ensemble des alcanes de formule CₙH₂ₙ₊₂ avec 8≤n≤12 ont un nombre d'atomes de carbone pair.

Dans un mode de réalisation, au moins un alcane de formule CₙH₂ₙ₊₂ avec n≤12 a un nombre d'atomes de carbone impair.

Dans un mode de réalisation, au moins un alcane de formule CₙH₂ₙ₊₂ avec 8≤n≤12 a un nombre d'atomes de carbone impair.

Dans un mode de réalisation, au moins un alcane de formule CₙH₂ₙ₊₂ avec n≤12 est choisi dans le groupe comprenant : le dodécane, 2-méthyle undécane (CAS 7045-71-8), 3-méthyle undécane (CAS 1002-43-3), 4-méthyle undécane (CAS 2980-69-0), 5-méthyle undécane (CAS 1632-70-8),6-méthyle undécane (CAS 17302-33-9), 2,4-diméthyle décane (CAS 2801-84-5) 4,4-diméthyle décane (CAS 17312-39-9), 3,5-diméthyle décane(17312-48-0), 2,5-diméthyle décane (CAS 17312-50-4), 2,3-diméthyle décane (17312-44-6), 3,3-diméthyle décane (17302-38-4), 3,7-diméthyle décane (CAS 17312-54-8), 3,4,6-triméthyle nonane (CAS 62184-24-1) 3,5,6-triméthyle nonane (CAS 62184-26-3), 3,5,7-triméthyle nonane (CAS 62184-27-4), 2,5,7-triméthyle nonane (CAS 62184-14-9), 2,5,6-triméthyle nonane (CAS 62184-13-8), 2,5,7-triméthyle nonane (CAS 62184-14-9), 2,5,8-triméthyle nonane (CAS 49557-09-7), 3,3,4,5-tétraméthyle octane (CAS 62185-21-1), 2,3,4,5-tétraméthyle octane (CAS 62199-27-3), 2,2,4,5-tétraméthyle octane (CAS 62183-80-6), 2,2,5,7-tétraméthyle octane (CAS 62199-19-3), 2,3,4,7-tétraméthyle octane (CAS 62199-29-5), 2,4,4,7-tétraméthyle octane (CAS 35866-96-7),3-éthyle-4-méthyle nonane (CAS 62184-45-6), 3-éthyle-4,5-diméthyle octane (CAS 62183-72-6), 2,5-diméthyle-6-éthyle octane (CAS 62183-50-0), undécane (CAS 1120-21-4), 2-méthyle décane ( CAS 6975-98-0), le 4-méthyle décane (CAS 2847-72-5), le 3-méthyle décane (13141-34-3), le 5-méthyle décane (CAS 13151-35-4), 2-6 diméthyle nonane (CAS 17302-28-2), 3,7-diméthyle nonane (CAS 17302-32-8), 4,5 diméthyle nonane (CAS 17302-23-7), 2,3-diméthyle nonane (CAS 2884-06-2), 2,4,6-triméthyle octane (CAS 62016-37-9), 2,5,6-triméthyle octane (CAS 62016-14-2), le décane (CAS 124-18-5), le 2-méthyle nonane (CAS 871-83-0), le 4-méthyle nonane (CAS 17301-94-9), le 3-méthyle nonane (CAS 5911-04-6), le 3-éthyle octane (CAS 5881-17-4), le 2,2-diméthyle octane (CAS 15869-87-1), le 2,3 dimethyle octane (CAS 7146-60-3), le 2,5- diméthyle octane (CAS 15869-89-3), le 3,5 diméthyle octane (CAS 15869-93-9), le 4-propyle heptane (CAS 3178-29-8), le 3-éthyle-2-méthyle heptane (CAS 14676-29-0), le 2,2,3-triméthyle heptane (CAS 52896-92-1), le 2,3,5 trimethyle heptane (CAS 20278-85-7), le 2,3,6-trimethyle heptane (CAS 4032-93-3), le 3,3,4-triméthyle heptane (CAS 20278-87-9), le 2,3,4 triméthyle heptane (CAS 52896-95-4), le 2,2,4 triméthyle heptane (CAS : 14720-74-2) le 3,3-diéthyle hexane (CAS 17302-02-2), le 2,2,3,3-tétraméthyle hexane (CAS 13475-81-5), le 3-éthyle-2,2,3-triméthyle pentane (CAS 52897-17-3), nonane (111-84-2), 2-méthyle octane (3221-61-2), 3-méthyle octane (CAS 2216-33-3), 4-méthyle octane (CAS 2216-34-4), 2,4-diméthyle heptane (CAS 2213-23-2), 2,6-diméthyle heptane (CAS 1072-05-5), 2,3-diméthyle heptane (CAS 3074-71-3), 2,5-diméthyle heptane (CAS 2216-30-0), 2,2-diméthyle heptane (CAS 1071-26-7), 2,2,5-triméthyle hexane (CAS 3522-94-9), 2,3,5-triméthyle hexane (CAS 1069-53-0), 2,2,4-triméthyle hexane (CAS 167476-26-5), 2,3,4 triméthyle hexane (CAS 921-47-1), 3-éthyle heptane (CAS 15869-80-4), 4-éthyle heptane (CAS 2216-32-2),octane (CAS 111-65-9), 2,2,4-triméthyle heptane (CAS 592-27-8), 3-méthyle heptane (CAS 589-81-1), 2-méthyle heptane (CAS 592-27-8), 2,3,4-triméthyle pentane (CAS 565-75-3), 2,4 diméthyle hexane (CAS 589-43-5), 2,5 diméthyle hexane (CAS 592-13-2), 3,4 diméthyle hexane (CAS 583-48-2), 3-éthyle hexane (CAS 619-99-8), 4-méthyle heptane (CAS 589-53-7), et leurs mélanges.

Dans un mode de réalisation, au moins un alcane de formule CₙH₂ₙ₊₂ avec n≤12 est linéaire.

Dans un mode de réalisation, l'ensemble des alcanes de formule CₙH₂ₙ₊₂ avec n≤12 sont linéaires.

Dans un mode de réalisation, au moins un alcane de formule CₙH₂ₙ₊₂ avec n≤12 est ramifié.

Dans un mode de réalisation, l'ensemble des alcanes de formule CₙH₂ₙ₊₂ avec n≤12 sont ramifiés.
Dans un mode de réalisation, le au moins un alcane de formule CₙH₂ₙ₊₂ avec n≤12 est choisi dans le groupe constitué par le dodécane, le undécane, décane, nonane et/ou un octane.

Dans un mode de réalisation, au moins un alcane de formule CₙH₂ₙ₊₂ avec n≤12 est un dodécane.

Dans un mode de réalisation, au moins un alcane de formule CₙH₂ₙ₊₂ avec n≤12 est un undécane.

Dans un mode de réalisation, au moins un alcane de formule CₙH₂ₙ₊₂ avec n≤12 est un décane.

Dans un mode de réalisation, au moins un alcane de formule CₙH₂ₙ₊₂ avec n≤12 est un nonane.

Dans un mode de réalisation, au moins un alcane de formule CₙH₂ₙ₊₂ avec n≤12 est un octane.

Dans un mode de réalisation, les alcanes de formule CₙH₂ₙ₊₂ avec n≤12 comprennent un mélange de décane et de dodécane.

Dans un mode de réalisation, les alcanes de formule CₙH₂ₙ₊₂avec n≤12 consistent en un mélange de décane et de dodécane.

Dans un mode de réalisation, au moins un alcane de formule CₙH₂ₙ₊₂ avec 8≤n≤12 est linéaire.

Dans un mode de réalisation, l'ensemble des alcanes de formule CₙH₂ₙ₊₂avec 8≤n≤12 sont linéaires.

Dans un mode de réalisation, au moins un alcane de formule CₙH₂ₙ₊₂ avec 8≤n≤12 est ramifié.

Dans un mode de réalisation, l'ensemble des alcanes de formule CₙH₂ₙ₊₂ avec 8≤n≤12 sont ramifiés.

Dans un mode de réalisation, le au moins un alcane de formule CₙH₂ₙ₊₂ avec 8≤n≤12 est choisi dans le groupe constitué par le dodécane, le undécane, décane, nonane et/ou un octane.

Dans un mode de réalisation, au moins un alcane de formule CₙH₂ₙ₊₂ avec 8≤n≤12 est un dodécane.

Dans un mode de réalisation, au moins un alcane de formule CₙH₂ₙ₊₂ avec 8≤n≤12 est un undécane.

Dans un mode de réalisation, au moins un alcane de formule CₙH₂ₙ₊₂ avec 8≤n≤12 est un décane.

Dans un mode de réalisation, au moins un alcane de formule CₙH₂ₙ₊₂ avec 8≤n≤12 est un nonane.

Dans un mode de réalisation, au moins un alcane de formule CₙH₂ₙ₊₂ avec 8≤n≤12 est un octane.

Dans un mode de réalisation, les alcanes de formule CₙH₂ₙ₊₂ avec 8≤n≤12 comprennent un mélange de décane et de dodécane.

Dans un mode de réalisation, les alcanes de formule CₙH₂ₙ₊₂ avec 8≤n≤12 consistent en un mélange de décane et de dodécane.

On appelle « indice de naturalité » d'une composition organique le pourcentage de carbone organique d'origine végétale déterminé par une analyse au radiocarbone (¹⁴C) selon l'une des normes suivantes ASTM D6866, EN 16640 ou EN 16785-1.

Au sens de la présente invention, la méthode OCDE 301F permet d'apprécier la biodégradabilité d'une substance par un essai de respirométrie manométrique.

Au sens de la présente invention, la méthode OCDE 301F permet d'apprécier la biodégradabilité d'une substance par un essai de respirométrie manométrique.

Dans un mode de réalisation, l'alcane de formule CₙH₂ₙ₊₂ avec n≤12 présente un indice de naturalité supérieur ou égal à 60% déterminé par la teneur en déterminé par la teneur en de carbone 14 (¹⁴C) selon l'une des normes suivantes ASTM D6866, EN 16640 ou EN 16785-1

Dans un mode de réalisation, l'alcane de formule CₙH₂ₙ₊₂ avec n≤12 présente un indice de naturalité supérieur ou égal à 70% déterminé par la teneur en carbone déterminé par la teneur en de carbone 14 (¹⁴C) selon l'une des normes suivantes ASTM D6866, EN 16640 ou EN 16785-1

Dans un mode de réalisation, l'alcane de formule CₙH₂ₙ₊₂ avec n≤12 présente un indice de naturalité supérieur ou égal à 75% déterminé par la teneur en de carbone 14 (¹⁴C) selon l'une des normes suivantes ASTM D6866, EN 16640 ou EN 16785-1

Dans un mode de réalisation, l'alcane de formule CₙH₂ₙ₊₂ avec n≤12 présente un indice de naturalité supérieur ou égal à 85% déterminé par la teneur en de carbone 14 (¹⁴C) selon l'une des normes suivantes ASTM D6866, EN 16640 ou EN 16785-1

Dans un mode de réalisation, l'alcane de formule CₙH₂ₙ₊₂ avec n≤12 présente un indice de naturalité supérieur ou égal à 90% déterminé par la teneur en de carbone 14 (¹⁴C) selon l'une des normes suivantes ASTM D6866, EN 16640 ou EN 16785-1

Avantageusement, l'alcane de formule CₙH₂ₙ₊₂ avec n≤12 présente un indice de naturalité de 100% déterminé par la teneur en de carbone 14 (¹⁴C) selon l'une des normes suivantes ASTM D6866, EN 16640 ou EN 16785-1

Dans un mode de réalisation, l'alcane de formule CₙH₂ₙ₊₂ avec n≤12 est obtenu à partir de matières premières d'origine végétale, bactérienne ou animale.

Dans un mode de réalisation, l'alcane de formule CₙH₂ₙ₊₂ avec n≤12 est obtenu à partir de matières premières d'origine végétale.

Dans un mode de réalisation, l'alcane de formule CₙH₂ₙ₊₂ avec n≤12 présente une biodégradabilité d'au moins 50% selon la méthode OCDE 301F.

Dans un mode de réalisation, l'alcane de formule CₙH₂ₙ₊₂ avec n≤12 présente une biodégradabilité d'au moins 60% selon la méthode OCDE 301F.

Dans un mode de réalisation, l'alcane de formule CₙH₂ₙ₊₂ avec n≤12 présente une biodégradabilité de 50 à 80% selon la méthode OCDE 301F.

Dans un mode de réalisation, l'alcane de formule CₙH₂ₙ₊₂ avec n≤12 présente une biodégradabilité de 50 à 70% selon la méthode OCDE 301F.

Dans un mode de réalisation, l'alcane de formule CₙH₂ₙ₊₂ avec n≤12 présente une biodégradabilité de 50 à 60% selon la méthode OCDE 301F.

Dans un mode de réalisation, l'alcane de formule CₙH₂ₙ₊₂ avec n≤12 présente une biodégradabilité de 60 à 70% selon la méthode OCDE 301F.

Dans un mode de réalisation, l'alcane de formule CₙH₂ₙ₊₂ avec n≤12 présente une biodégradabilité de 70 à 80% selon la méthode OCDE 301F.

Dans un mode de réalisation, l'alcane de formule CₙH₂ₙ₊₂ avec 8≤n≤12 présente un indice de naturalité supérieur ou égal à 60% déterminé par la teneur en de carbone 14 (¹⁴C) selon l'une des normes suivantes ASTM D6866, EN 16640 ou EN 16785-1

Dans un mode de réalisation, l'alcane de formule CₙH₂ₙ₊₂ avec 8≤n≤12 présente un indice de naturalité supérieur ou égal à 70% déterminé par la teneur en de carbone 14 (¹⁴C) selon l'une des normes suivantes ASTM D6866, EN 16640 ou EN 16785-1

Dans un mode de réalisation, l'alcane de formule CₙH₂ₙ₊₂ avec 8≤n≤12 présente un indice de naturalité supérieur ou égal à 75% déterminé par la teneur en de carbone 14 (¹⁴C) selon l'une des normes suivantes ASTM D6866, EN 16640 ou EN 16785-1

Dans un mode de réalisation, l'alcane de formule CₙH₂ₙ₊₂ avec 8≤n≤12 présente un indice de naturalité supérieur ou égal à 85% déterminé par la teneur en de carbone 14 (¹⁴C) selon l'une des normes suivantes ASTM D6866, EN 16640 ou EN 16785-1

Dans un mode de réalisation, l'alcane de formule CₙH₂ₙ₊₂ avec 8≤n≤12 présente un indice de naturalité supérieur ou égal à 90% déterminé par la teneur en de carbone 14 (¹⁴C) selon l'une des normes suivantes ASTM D6866, EN 16640 ou EN 16785-1

Avantageusement, l'alcane de formule CₙH₂ₙ₊₂ avec 8≤n≤12 présente un indice de naturalité de 100% déterminé par la teneur en de carbone 14 (¹⁴C) selon l'une des normes suivantes ASTM D6866, EN 16640 ou EN 16785-1

Dans un mode de réalisation, l'alcane de formule CₙH₂ₙ₊₂ avec 8≤n≤12 est obtenu à partir de matières premières d'origine végétale, bactérienne ou animale.

Dans un mode de réalisation, l'alcane de formule CₙH₂ₙ₊₂ avec 8≤n≤12 est obtenu à partir de matières premières d'origine végétale.

Dans un mode de réalisation, l'alcane de formule CₙH₂ₙ₊₂ avec 8≤n≤12 présente une biodégradabilité d'au moins 50% selon la méthode OCDE 301F.

Dans un mode de réalisation, l'alcane de formule CₙH₂ₙ₊₂ avec 8≤n≤12 présente une biodégradabilité d'au moins 60% selon la méthode OCDE 301F.

Dans un mode de réalisation, l'alcane de formule CₙH₂ₙ₊₂ avec 8≤n≤12 présente une biodégradabilité de 50 à 80% selon la méthode OCDE 301F.

Dans un mode de réalisation, l'alcane de formule CₙH₂ₙ₊₂ avec 8≤n≤12 présente une biodégradabilité de 50 à 70% selon la méthode OCDE 301F.

Dans un mode de réalisation, l'alcane de formule CₙH₂ₙ₊₂ avec 8≤n≤12 présente une biodégradabilité de 50 à 60% selon la méthode OCDE 301F.

Dans un mode de réalisation, l'alcane de formule CₙH₂ₙ₊₂ avec 8≤n≤12 présente une biodégradabilité de 60 à 70% selon la méthode OCDE 301F.

Dans un mode de réalisation, l'alcane de formule CₙH₂ₙ₊₂ avec 8≤n≤12 présente une biodégradabilité de 70 à 80% selon la méthode OCDE 301F.

Dans un mode de réalisation, la composition selon l'invention comprend au moins un oxo-ester de formule II : 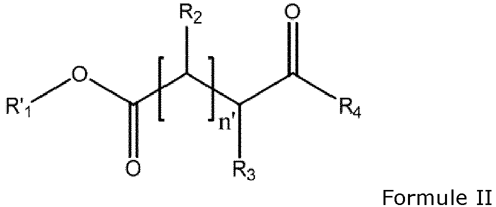 dans laquelle,
- R'₁ est choisi dans le groupe constitué par les alkyles linéaires ou ramifiés comprenant de 1 à 4 atomes de carbone ;
- R₂, R₃ et R₄ identiques ou différents sont choisis dans le groupe constitué par l'atome d'hydrogène ou les alkyles linéaires ou ramifiés comprenant de 1 à 4 atomes de carbone; et
- n'est un entier naturel compris entre 1 et 4.

Dans un mode de réalisation, la composition selon l'invention comprend au moins un oxo-ester de formule II, dans laquelle R₂ et R₃ identiques ou différents sont choisis dans le groupe constitué par l'atome d'hydrogène, le groupement méthyl ou le groupement éthyl, R₄ est choisi dans le groupe constitué par les alkyls linéaires ou ramifiés comprenant de 1 à 3 atomes de carbone et n'est un entier naturel compris entre 1 et 3.

Dans un mode de réalisation, la composition selon l'invention comprend au moins un oxo-ester de formule I dans laquelle R₂ et R₃ sont un atome d'hydrogène, R4 est un groupement méthyl et n'est égal à 1.

Dans un mode de réalisation, la composition selon l'invention comprend au moins un oxo-ester de formule II dans laquelle R₂ et R₃ sont un atome d'hydrogène, R4 est un groupement méthyl et n'est égal à 1.

Dans un mode de réalisation, la composition selon l'invention comprend un oxo-ester seul ou en mélange.

Dans un mode de réalisation, l'oxo-ester seul ou en mélange est choisi dans le groupe comprenant : le lévulinate de méthyle (CAS 624-45-3), le lévulinate d'éthyle (CAS 539-88-8), le lévulinate de propyle (645-67-0), le lévulinate d'isopropyle (CAS 21884-26-4), le lévulinate de butyle (CAS 2052-15-5), le lévulinate d'isobutyle (CAS 3757-32-2), le lévulinate de tert-butyle (CAS 2854-10-6), le lévulinate de s-butyle (CAS 85734-01-6), le lévulinate de penthyl (CAS 20279-49-6) le lévulinate d'hexyle (CAS 24431-34-3), le lévulinate d'octyl (CAS 41780-57-8), l'ester éthylique de l'acide 2-méthyl-4-oxovalérique (CAS 4749-12-6), le 6-oxoheptanoate de méthyle (CAS 2046-21-1), le 4-oxohexanoate de méthyle (CAS 2955-62-6), le 5-oxohexanoate de méthyle (CAS 13984-50-4), le 3-méthyl -5-oxohexanoate de méthyle (CAS 14983-18-7), l'ester méthylique de l'acide 5-cétoénanthique (17745-32-3), l'ester méthylique (CAS 36045-56-4), l'acide pentanoïque, 2-éthyl-4-oxo-méthyl ester (CAS 62359-06-2), le 3-méthyl-4-oxohexanoate de méthyle (CAS 69448-35-7), l'acide hexanoïque, 2-méthyl-5-oxo méthyl ester (CAS 38872-30-9), l'acide pentanoïque, 3-méthyl-4-oxo éthyl ester (CAS 55424-74-3), l'ester 2-éthyl-4-oxométhylique de l'acide hexanoïque (CAS 75436-59-8), l'ester 2,4-diméthyl-5-oxométhylique de l'acide hexanoïque (CAS 93176-58-0), l'ester 4-oxo-2-propyl-méthylique de l'acide pentanoïque (CAS 244196-06-3), l'ester 2,5-diméthyl-4-oxométhylique de l'acide hexanoïque (CAS 1249353-11-4), l'ester 4-oxométhylique de l'acide octanoïque (CAS : 4316-48-7), l'ester 2-méthyl-6-oxo-méthylique de l'acide heptanoïque (CAS 2570-90-3), l'ester 3-méthyl-6-oxo-méthylique de l'acide heptanoïque (CAS 5128-55-2), l'ester 6-méthyl-5-oxo-méthylique de l'acide heptanoïque (CAS 23575-33-9), l'ester 3-méthyl-5-oxo-éthylique de l'acide hexanoïque (CAS 38052-21-0), l'ester 2-méthyl-5-oxo méthylique de l'acide heptanoïque (CAS 25912-38-3), l'ester 4-méthyl-6-oxo méthylique de l'acide heptanoïque (CAS 41841-53-6), l'ester 5-méthyl-4-oxo méthylique de l'acide heptanoïque (CAS 42511-74-0), l'ester 4-méthyl-5-oxo méthylique de l'acide heptanoïque (CAS 54225-40-0), l'ester 3,5-diméthyl-4-oxo méthylique de l'acide hexanoïque (CAS 64712-02-3), l'ester 6-méthyl-4-oxo méthylique de l'acide heptanoïque (CAS 76678-33-6), l'acide heptanoïque, l'ester méthylique de 2-méthyl-4-oxo- (CAS 90647-21-5), l'acide hexanoïque, ester méthylique de 4-éthyl-5-oxo (CAS 90647-24-8), acide heptanoïque, ester méthylique de 3-méthyl-5-oxo (CAS 103252-99-9), l'acide hexanoïque, 2-éthyl-5-oxo-, ester méthylique (CAS 103260-39-5), l'acide hexanoïque 3 acétyl ester méthylique (CAS 1081559-93-4), l'acide heptanoïque 5 méthyl-6-oxo ester méthylique (CAS 344295-02-9), l'ester 3-méthyl-4-oxo méthylique de l'acide heptanoïque (CAS 64712-01-2), le 2-(1méthyléthyl)-4-oxopentaoate de méthyle (CAS 99183-33-2), le 2,3-diméthyl-4-oxohexanoate de méthyle (CAS 86044-19-1), le 2 éthyl-4-oxopentanoate d'éthyle (CAS 101514-30-1), l'ester éthylique 5-oxo de l'acide hexanoïque (CAS 13984-57-1), le 3-méthyl-4-oxohexanoate d'éthyle (CAS 42895-72-7), 2,3-diméthyl-4-oxopentanoate d'éthyle (CAS 136964-44-8), l'ester éthylique 4-oxo de l'acide hexanoïque (CAS 3249-33-0), l'ester 6-oxo-éthylique de l'acide heptanoïque (CAS 30956-41-3), le 4-oxohexanoate de 1-méthyléthyle (CAS 939422-07-8), l'ester 2-éthyl-4-oxo-éthylique de l'acide pentanoïque (CAS 101514-30-1), l'ester 2-éthyl-5-méthyl-4-oxo-méthylique de l'acide hexanoïque (CAS 1195311-69-3).

Dans un mode de réalisation, au moins un oxo-ester est un lévulinate.

Dans un mode de réalisation, au moins un oxo-ester est un lévulinate choisi dans le groupe comprenant : le lévulinate de méthyle (CAS 624-45-3), le lévulinate d'éthyle (CAS 539-88-8), le lévulinate de propyle (645-67-0), le lévulinate d'isopropyle (CAS 21884-26-4), le lévulinate de butyle (CAS 2052-15-5), le lévulinate d'isobutyle (CAS 3757-32-2), le lévulinate de tert-butyle (CAS 2854-10-6), le lévulinate de s-butyle (CAS 85734-01-6), le lévulinate de penthyl (CAS 20279-49-6) le lévulinate d'hexyle (CAS 24431-34-3), le lévulinate d'octyl (CAS 41780-57-8), ou leurs mélanges.

Dans un mode de réalisation, au moins un oxo-ester est un lévulinate de méthyle.

Dans un mode de réalisation, au moins un oxo-ester est un lévulinate de butyle.

Dans un mode de réalisation, au moins un oxo-ester est un lévulinate d'éthyle.

Dans un mode de réalisation, au moins un oxo-ester est un lévulinate de butyle.et/ou lévulinate d'éthyle.

Dans un mode de réalisation, l'oxo-ester comprend un lévulinate d'éthyle et/ou un lévulinate de butyle.

Dans un mode de réalisation, l'oxo-ester consiste en un lévulinate d'éthyle et/ou un lévulinate de butyle.

Dans un mode de réalisation, l'oxo-ester à une masse moléculaire inférieure ou égale à 200g/mol.

Dans un mode de réalisation, l'oxo-ester à une masse moléculaire inférieure ou égale à 180 g/mol.

Dans un mode de réalisation, l'oxo-ester à une masse moléculaire inférieure ou égale à 160 g/mol.

Dans un mode de réalisation, l'oxo-ester à une masse moléculaire inférieure ou égale à 150 g/mol.

Dans un mode de réalisation, la composition selon l'invention comprend un décane et un lévulinate d'éthyle.

Dans un mode de réalisation, la composition selon l'invention comprend un dodécane et un lévulinate d'éthyle.

Dans un mode de réalisation, la composition selon l'invention comprend un décane, un dodécane et un lévulinate d'éthyle.

Dans un mode de réalisation, la composition selon l'invention comprend un alcane ramifié de 10 atomes de carbones et un lévulinate d'éthyle.

Dans un mode de réalisation, la composition selon l'invention comprend un alcane ramifié de 12 atomes de carbones et un lévulinate d'éthyle.

Dans un mode de réalisation, la composition selon l'invention comprend un décane et un lévulinate de butyle.

Dans un mode de réalisation, la composition selon l'invention comprend un dodécane et un lévulinate de butyle.

Dans un mode de réalisation, la composition selon l'invention comprend un décane, un dodécane et un lévulinate de butyle.

Dans un mode de réalisation, la composition selon l'invention comprend un alcane ramifié de 10 atomes de carbones et un lévulinate de butyle.

Dans un mode de réalisation, la composition selon l'invention comprend un alcane ramifié de 12 atomes de carbones et un lévulinate de butyle.

Dans un mode de réalisation, la composition selon l'invention comprend un décane, un dodécane, un lévulinate d'éthyle et un lévulinate de butyle.

Dans un mode de réalisation, l'oxo-ester présente un indice de naturalité supérieur ou égal à 60% déterminé par la teneur en de carbone 14 (¹⁴C) selon l'une des normes suivantes ASTM D6866, EN 16640 ou EN 16785-1

Dans un mode de réalisation, l'oxo-ester présente un indice de naturalité supérieur ou égal à 70% déterminé par la teneur en de carbone 14 (¹⁴C) selon l'une des normes suivantes ASTM D6866, EN 16640 ou EN 16785-1

Dans un mode de réalisation, l'oxo-ester présente un indice de naturalité supérieur ou égal à 75% déterminé par la teneur en de carbone 14 (¹⁴C) selon l'une des normes suivantes ASTM D6866, EN 16640 ou EN 16785-1

Dans un mode de réalisation, l'oxo-ester présente un indice de naturalité supérieur ou égal à 85% déterminé par la teneur en de carbone 14 (¹⁴C) selon l'une des normes suivantes ASTM D6866, EN 16640 ou EN 16785-1

Dans un mode de réalisation, l'oxo-ester présente un indice de naturalité supérieur ou égal à 90% déterminé par la teneur en de carbone 14 (¹⁴C) selon l'une des normes suivantes ASTM D6866, EN 16640 ou EN 16785-1

Avantageusement, l'oxo-ester présente un indice de naturalité de 100% déterminé par la teneur en de carbone 14 (¹⁴C) selon l'une des normes suivantes ASTM D6866, EN 16640 ou EN 16785-1

Dans un mode de réalisation, l'oxo-ester est obtenu à partir de matières premières d'origine végétale, bactérienne ou animale.

Dans un mode de réalisation, le lévulinate est obtenu est issu de la biomasse.

Dans un mode de réalisation, l'oxo-ester présente une biodégradabilité d'au moins 50% selon la méthode OCDE 301F.

Dans un mode de réalisation, l'oxo-ester présente une biodégradabilité d'au moins 60% selon la méthode OCDE 301F.

Dans un mode de réalisation, l'oxo-ester présente une biodégradabilité de 50% à 80% selon la méthode OCDE 301F.

Dans un mode de réalisation, l'oxo-ester présente une biodégradabilité de 50% à 70% selon la méthode OCDE 301F.

Dans un mode de réalisation, l'oxo-ester présente une biodégradabilité de 50% à 60% selon la méthode OCDE 301F.

Dans un mode de réalisation, l'oxo-ester présente une biodégradabilité de 60 à 70% selon la méthode OCDE 301F.

Dans un mode de réalisation, l'oxo-ester présente une biodégradabilité de 70 à 80% selon la méthode OCDE 301F.

Dans un mode de réalisation, la composition selon l'invention comprend une teneur en un oxo-ester de formule I, de 10% à 50% (v/v), en volume par rapport au volume total de la composition selon l'invention.

Dans un mode de réalisation, la composition selon l'invention comprend une teneur en un oxo-ester de formule I, de 10% à 30% (v/v), par rapport au volume total de la composition selon l'invention.

Dans un mode de réalisation, la composition selon l'invention comprend une teneur en un oxo-ester de formule I, de 10% à 20% (v/v), par rapport au volume total de la composition selon l'invention.

Dans un mode de réalisation, la composition selon l'invention comprend une teneur en lévulinate de butyle d'au moins 1% (v/v), en volume par rapport au volume total de la composition selon l'invention.

Dans un mode de réalisation, la composition selon l'invention comprend une teneur en lévulinate de butyle d'au moins 5% (v/v), en volume par rapport au volume total de la composition selon l'invention.

Dans un mode de réalisation, la composition selon l'invention comprend une teneur en lévulinate de butyle de 1 à 60% (v/v), en volume par rapport au volume total de la composition selon l'invention.

Dans un mode de réalisation, la composition selon l'invention comprend une teneur en lévulinate de butyle de 5 à 25% (v/v), en volume par rapport au volume total de la composition selon l'invention.

Dans un mode de réalisation, la composition selon l'invention comprend une teneur en lévulinate de butyle d'environ 5% (v/v), en volume par rapport au volume total de la composition selon l'invention.

Dans un mode de réalisation, la composition selon l'invention comprend une teneur en lévulinate d'éthyle d'au moins 1% (v/v), en volume par rapport au volume total de la composition selon l'invention.

Dans un mode de réalisation, la composition selon l'invention comprend une teneur en lévulinate d'éthyle d'au moins 5% (v/v), en volume par rapport au volume total de la composition selon l'invention.

Dans un mode de réalisation, la composition selon l'invention comprend une teneur en lévulinate d'éthyle de 1% à 60% (v/v), en volume par rapport au volume total de la composition selon l'invention.

Dans un mode de réalisation, la composition selon l'invention comprend une teneur en lévulinate d'éthyle de 5% à 25% (v/v), en volume par rapport au volume total de la composition selon l'invention.

Dans un mode de réalisation, la composition selon l'invention comprend une teneur en lévulinate d'éthyle d'environ 5% (v/v), en volume par rapport au volume total de la composition selon l'invention.

Dans un mode de réalisation, la composition selon l'invention comprend une teneur en alcane de formule CₙH₂ₙ₊₂ avec n≤12 de 50% à 90% (v/v), en volume par rapport au volume total de la composition selon l'invention.

Dans un mode de réalisation, la composition selon l'invention comprend une teneur en alcane de formule CₙH₂ₙ₊₂ avec n≤12 de 70% à 90% (v/v), par rapport au volume total de la composition selon l'invention.

Dans un mode de réalisation, la composition selon l'invention comprend une teneur en alcane de formule CₙH₂ₙ₊₂ avec n≤12 de 80% à 90% (v/v), par rapport au volume total de la composition selon l'invention.

Dans un mode de réalisation, la composition selon l'invention comprend une teneur en alcane de formule CₙH₂ₙ₊₂ avec 8≤n≤12 de 50% à 90% (v/v), en volume par rapport au volume total de la composition selon l'invention.

Dans un mode de réalisation, la composition selon l'invention comprend une teneur en alcane de formule CₙH₂ₙ₊₂ avec 8≤n≤12 de 70% à 90% (v/v), par rapport au volume total de la composition selon l'invention.

Dans un mode de réalisation, la composition selon l'invention comprend une teneur en alcane de formule CₙH₂ₙ₊₂ avec 8≤n≤12 de 80% à 90% (v/v), par rapport au volume total de la composition selon l'invention.

Dans un mode de réalisation, la composition selon l'invention comprend une teneur en décane d'au moins 1% (v/v) en volume par rapport au volume total de la composition selon l'invention.

Dans un mode de réalisation, la composition selon l'invention comprend une teneur en décane d'au moins 5% (v/v) en volume par rapport au volume total de la composition selon l'invention.

Dans un mode de réalisation, la composition selon l'invention comprend une teneur en décane de 1% à 50% (v/v) en volume par rapport au volume total de la composition selon l'invention.

Dans un mode de réalisation, la composition selon l'invention comprend une teneur en décane de 5% à 30% (v/v) en volume par rapport au volume total de la composition selon l'invention.

Dans un mode de réalisation, la composition selon l'invention comprend une teneur en décane de 7,5% à 20% (v/v) en volume par rapport au volume total de la composition selon l'invention.

Dans un mode de réalisation, la composition selon l'invention comprend une teneur en dodécane d'au moins 20% (v/v) en volume par rapport au volume total de la composition selon l'invention.

Dans un mode de réalisation, la composition selon l'invention comprend une teneur en dodécane d'au moins 30% (v/v) en volume par rapport au volume total de la composition selon l'invention.

Dans un mode de réalisation, la composition selon l'invention comprend une teneur en dodécane de 30% à 80% (v/v) en volume par rapport au volume total de la composition selon l'invention.

Dans un mode de réalisation, la composition selon l'invention comprend une teneur en dodécane de 40% à 75% (v/v) en volume par rapport au volume total de la composition selon l'invention.

Dans un mode de réalisation, la composition selon l'invention comprend une proportion de décane de 5 % à 30 % (v/v) en volume par rapport au volume total des alcanes de formule CₙH₂ₙ₊₂ avec n≤12.

Dans un mode de réalisation, la composition selon l'invention comprend une proportion de décane de 10 % à 20 % (v/v) en volume par rapport au volume total des alcanes de formule CₙH₂ₙ₊₂ avec n≤12.

Dans un mode de réalisation, la composition selon l'invention comprend une proportion de dodécane de 70 % à 95 % (v/v) en volume par rapport au volume total des alcanes de formule CₙH₂ₙ₊₂ avec n≤12.

Dans un mode de réalisation, la composition selon l'invention comprend une proportion de dodécane de 80 % à 90 % (v/v) en volume par rapport au volume total des alcanes de formule CₙH₂ₙ₊₂ avec n≤12.

Dans un mode de réalisation, la composition selon l'invention comprend une proportion de décane de 5 % à 30 % (v/v) en volume par rapport au volume total des alcanes de formule CₙH₂ₙ₊₂ avec 8≤n≤12.

Dans un mode de réalisation, la composition selon l'invention comprend une proportion de décane de 10 % à 20 % (v/v) en volume par rapport au volume total des alcanes de formule CₙH₂ₙ₊₂ avec 8≤n≤12.

Dans un mode de réalisation, la composition selon l'invention comprend une proportion de dodécane de 70 % à 95 % (v/v) en volume par rapport au volume total des alcanes de formule CₙH₂ₙ₊₂ avec 8≤n≤12.

Dans un mode de réalisation, la composition selon l'invention comprend une proportion de dodécane de 80 % à 90 % (v/v) en volume par rapport au volume total des alcanes de formule CₙH₂ₙ₊₂ avec 8≤n≤12.

Dans un mode de réalisation, la composition selon l'invention ne comprend pas de dérivés pétrochimiques.

Dans un mode de réalisation, la composition ne comprend pas de karité et/ou d'extrait de karité.

Dans un mode de réalisation, la composition selon l'invention comprend en outre au moins une huile.

Un autre objet selon la présente invention concerne donc une composition caractérisée en ce qu'elle comprend :
a) au moins un alcane de formule CₙH₂ₙ₊₂ avec n≤ 12, à une teneur de 40% à 95% (v/v) en volume par rapport au volume total de la composition et
   au moins un oxo-ester de formule I : 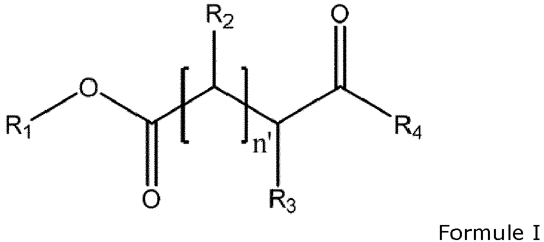 dans laquelle,
   - R₁ est choisi dans le groupe constitué par les alkyles linéaires ou ramifiés comprenant de 1 à 8 atomes de carbone ;
   - R₂, R₃ et R₄ identiques ou différents sont choisis dans le groupe constitué par l'atome d'hydrogène et/ou les alkyles linéaires ou ramifiés comprenant de 1 à 4 atomes de carbone; et
   - n'est un entier naturel de 1 à 4
      à une teneur de 5% à 60% (v/v) en volume par rapport au volume total de la composition selon l'invention.
b) au moins une huile.

Un autre objet selon la présente invention concerne donc une composition caractérisée en ce qu'elle comprend :
a) au moins un alcane de formule CₙH₂ₙ₊₂ avec 8≤n≤12, à une teneur de 40% à 95% (v/v) en volume par rapport au volume total de la composition et
b) au moins un oxo-ester de formule I : 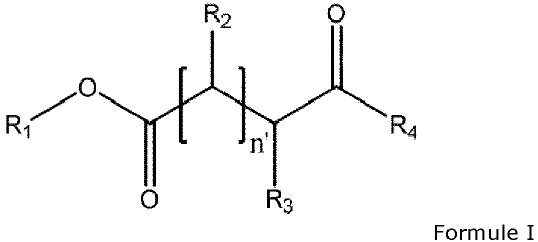 dans laquelle,
   - R₁ est choisi dans le groupe constitué par les alkyles linéaires ou ramifiés comprenant de 1 à 8 atomes de carbone ;
   - R₂, R₃ et R₄ identiques ou différents sont choisis dans le groupe constitué par l'atome d'hydrogène et/ou les alkyles linéaires ou ramifiés comprenant de 1 à 4 atomes de carbone; et
   - n'est un entier naturel de 1 à 4
      à une teneur de 5% à 60% (v/v) en volume par rapport au volume total de la composition selon l'invention.
c) au moins une huile.
Dans un mode de réalisation de la composition comprenant au moins une huile, l'au
moins un oxo-ester est choisi parmi les oxo-esters de formule II : 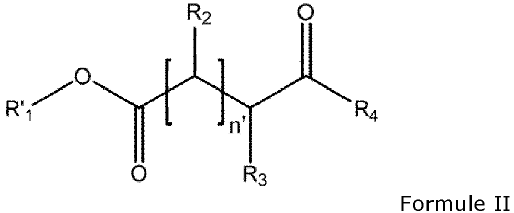 dans laquelle,
- R'₁ est choisi dans le groupe constitué par les alkyles linéaires ou ramifiés comprenant de 1 à 4 atomes de carbone ;
- R₂, R₃ et R₄ identiques ou différents sont choisis dans le groupe constitué par l'atome d'hydrogène ou les alkyles linéaires ou ramifiés comprenant de 1 à 4 atomes de carbone; et
- n'est un entier naturel compris entre 1 et 4.

Dans un mode de réalisation de la composition comprenant au moins une huile, l'au moins un oxo-ester est choisi parmi les oxo-esters de formule I dans laquelle R₂ et R₃ sont un atome d'hydrogène, R₄ est un groupement méthyl et n'est égal à 1.

Dans un mode de réalisation de la composition comprenant au moins une huile, l'au moins un oxo-ester est choisi parmi les oxo-esters de formule II, dans laquelle R₂ et R₃ identiques ou différents sont choisis dans le groupe constitué par l'atome d'hydrogène, le groupement méthyl ou le groupement éthyl, R₄ est choisi dans le groupe constitué par les alkyls linéaires ou ramifiés comprenant de 1 à 3 atomes de carbone et n'est un entier naturel compris entre 1 et 3.

Dans un mode de réalisation de la composition comprenant au moins une huile, l'au moins un oxo-ester est choisi parmi les oxo-esters de formule II dans laquelle R₂ et R₃ sont un atome d'hydrogène, R4 est un groupement méthyl et n'est égal à 1.

La présence d'une huile dans la composition présente l'avantage d'apporter des propriétés lubrifiantes à la composition. Elle présente également l'avantage de déposer un film protecteur sur la surface, permettant d'éviter l'encrassement. La présence d'huile permet enfin d'augmenter le pouvoir dégraissant de la composition.

Dans un mode de réalisation, l'huile comprend une huile d'origine végétale et/ou synthétique.

Dans un mode de réalisation, la composition selon l'invention ne comprend pas d'huile minérale.

Dans un mode de réalisation, l'huile comprend une huile hydrocarbonée, siliconée, fluorée et/ou fluorosiliconée.

Au sens de la présente invention, on entend par huile hydrocarbonée une huile essentiellement constituée d'atomes de carbone, d'hydrogène et éventuellement d'atomes d'oxygène et ou d'azote.

Dans un mode de réalisation, ces huiles hydrocarbonées sont choisies dans le groupe des alcanes linéaires ou ramifiés d'origine minérale ou synthétique comme les alcanes linéaires ou ramifiés de 17 à 40 atomes de carbone, les (poly)esters et (poly)éthers, et en particulier les (poly)esters d'acides en C₂-C₂₄ (de préférence en C₆-C₂₀), les triglycérides d'acides gras en C₆-C₂₀, les huiles végétales, les di-alkyles carbonates, les acides gras ramifiées et/ou insaturés et/ou les alcools gras ramifiés et/ou insaturés et leurs mélanges.

Dans un mode de réalisation, les huiles siliconées elles sont choisies dans le groupe comprenant les huiles silicones phénylées, les polydiméthylsiloxanes non volatiles, ou dérivés de polydiméthylsiloxanes non volatiles et leurs mélanges.

Dans un mode de réalisation, l'huile comprend une huile choisie dans le groupe constitué des alcanes linéaires ou ramifiés non volatils.

Dans un mode de réalisation, les alcanes linéaires ou ramifiés non volatils sont choisis dans le groupe constitué des alcanes linéaires ou ramifiés de 17 à 40 atomes de carbone.

Dans un mode de réalisation, l'huile comprend une huile d'origine végétale.

Dans un mode de réalisation, l'huile comprend une huile hydrocarbonée végétale.

Dans un mode de réalisation, l'huile comprend une huile hydrocarbonée végétale choisies dans le groupe comprenant les huiles de germe de blés, de tournesol, de pépins de raisin, de sésame, de coco, d'abricot, de maïs, d'avocat, de soja, d'olive, d'huile d'amande douce, de coton, de palme, de macadamia, de colza, de jojoba, de noisette, de pavot, de luzerne, de potimarron, de cassis, de courge, d'onagre, d'orge, et leurs mélanges.

Dans un mode de réalisation, la composition selon l'invention ne comprend pas d'huile de ricin.

Dans un mode de réalisation, l'huile comprend une huile de coco.

Dans un mode de réalisation, l'huile consiste en une huile de coco.

Dans un mode de réalisation, la composition selon l'invention comprend une teneur en huile inférieure ou égale à 30% (v/v) en volume d'huile par rapport au volume total de la composition selon l'invention.

Dans un mode de réalisation, la composition selon l'invention comprend une teneur en huile inférieure ou égale à 20% (v/v) en volume d'huile par rapport au volume total de la composition selon l'invention.

Dans un mode de réalisation, la composition selon l'invention comprend une teneur en huile inférieure ou égale à 15% (v/v) en volume d'huile par rapport au volume total de la composition selon l'invention.

Dans un mode de réalisation, la composition selon l'invention comprend une teneur en huile inférieure de 5 à 20% (v/v) en volume d'huile par rapport au volume total de la composition selon l'invention.

Dans un mode de réalisation, la composition selon l'invention présente un indice de naturalité supérieur ou égal à 60% déterminé par la teneur en de carbone 14 (¹⁴C) selon l'une des normes suivantes ASTM D6866, EN 16640 ou EN 16785-1

Dans un mode de réalisation, la composition selon l'invention présente un indice de naturalité supérieur ou égal à 70% déterminé par la teneur en de carbone 14 (¹⁴C) selon l'une des normes suivantes ASTM D6866, EN 16640 ou EN 16785-1

Dans un mode de réalisation, la composition selon l'invention présente un indice de naturalité supérieur ou égal à 75% déterminé par la teneur en de carbone 14 (¹⁴C) selon l'une des normes suivantes ASTM D6866, EN 16640 ou EN 16785-1

Dans un mode de réalisation, la composition selon l'invention présente un indice de naturalité supérieur ou égal à 85% déterminé par la teneur en de carbone 14 (¹⁴C) selon l'une des normes suivantes ASTM D6866, EN 16640 ou EN 16785-1

Dans un mode de réalisation, la composition selon l'invention présente un indice de naturalité supérieur ou égal à 90% déterminé par la teneur en de carbone 14 (¹⁴C) selon l'une des normes suivantes ASTM D6866, EN 16640 ou EN 16785-1

Avantageusement, la composition selon l'invention présente un indice de naturalité de 100% déterminé par la teneur en de carbone 14 (¹⁴C) selon l'une des normes suivantes ASTM D6866, EN 16640 ou EN 16785-1

Dans un mode de réalisation, la composition selon l'invention est obtenue à partir de matières premières d'origine végétale, bactérienne ou animale.

Dans un mode de réalisation, la composition selon l'invention présente une biodégradabilité d'au moins 50% selon la méthode OCDE 301F.

Dans un mode de réalisation, la composition selon l'invention présente une biodégradabilité d'au moins 60% selon la méthode OCDE 301F.

Dans un mode de réalisation, la composition selon l'invention présente une biodégradabilité de 50% à 80% selon la méthode OCDE 301F.

Dans un mode de réalisation, la composition selon l'invention présente une biodégradabilité de 50% à 70% selon la méthode OCDE 301F.

Dans un mode de réalisation, la composition selon l'invention présente une biodégradabilité de 50% à 60% selon la méthode OCDE 301F.

Dans un mode de réalisation, la composition selon l'invention présente une biodégradabilité de 60% à 70% selon la méthode OCDE 301F.

Dans un mode de réalisation, la composition selon l'invention présente une biodégradabilité de 70% à 80% selon la méthode OCDE 301F.

Dans un mode de réalisation, la composition selon l'invention présente une densité de 0,6 à 1 g/cm3.

Dans un mode de réalisation, la composition selon l'invention présente une densité de 0,75 à 0,85 g/cm3.

Dans un mode de réalisation, la composition selon l'invention présente une viscosité dynamique de 1 à 3 centipoises.

Dans un mode de réalisation, la composition selon l'invention présente une viscosité dynamique de 1,5 à 2,75 centipoises.

Dans un mode de réalisation, la composition selon l'invention présente une viscosité cinématique, de 1,5 à 3 centistokes.

Dans un mode de réalisation, la composition selon l'invention présente une viscosité cinématique de 1,8 à 2,6 centistokes.

L'invention concerne également un aérosol comprenant la composition selon l'invention et un gaz propulseur.

Dans un mode de réalisation, le gaz propulseur est choisi dans le groupe du dioxyde de carbone (COz), du butane, du propane, de l'isobutane, du diméthyle éther, de l'azote, du protoxyde d'azote ou leurs mélanges.

Dans un mode de réalisation, le gaz propulseur est choisi dans le groupe du dioxyde de carbone (COz), du butane, du propane ou de leurs mélanges.

Dans un mode de réalisation, le gaz propulseur est du dioxyde de carbone.

Dans un mode de réalisation, l'aérosol selon l'invention est conditionné dans une bombe aérosol.

Dans un mode de réalisation, la pression intérieure de la bombe aérosol est d'au moins 7 bars.

Dans un mode de réalisation, la pression intérieure de la bombe aérosol est d'au moins 8 bars.

Dans un mode de réalisation, la bombe aérosol comprend une buse permettant une diffusion en brume large d'au moins 20 cm.

Dans un mode de réalisation, la bombe aérosol comprend une buse permettant une diffusion en brume large d'au moins 30 cm.

Dans un mode de réalisation, la bombe aérosol permet un débit d'au moins 1 gramme par seconde.

Dans un mode de réalisation, la bombe aérosol permet un débit d'au moins 2 grammes par seconde.

Dans un mode de réalisation, la bombe aérosol permet un débit d'au moins 4 grammes par seconde.

Un autre objet selon la présente invention concerne un procédé pour dégraisser, nettoyer, protéger, dégripper, faire briller et/ou lubrifier une surface d'un objet comprenant au moins une étape d'application sur ladite surface d'une composition comprenant :
a) Au moins un alcane de formule CₙH₂ₙ₊₂ avec n≤ 12, et
   Au moins un oxo-ester de formule I, 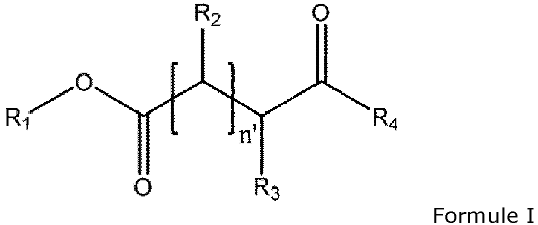 dans laquelle,
   - R₁ est choisi dans le groupe constitué par les alkyles linéaires ou ramifiés comprenant de 1 à 8 atomes de carbone ;
   - R₂, R₃ et R₄ identiques ou différents sont choisis dans le groupe constitué par l'atome d'hydrogène et/ou les alkyles linéaires ou ramifiés comprenant de 1 à 4 atomes de carbone; et
   - n'est un entier naturel de 1 à 4.

Dans un mode de réalisation, le procédé pour dégraisser, nettoyer, protéger, dégripper, faire briller et/ou lubrifier une surface d'un objet selon l'invention comprend au moins une étape d'application sur ladite surface d'une composition comprenant :
a) Au moins un alcane de formule CₙH₂ₙ₊₂ avec 8≤n≤12, et
b) Au moins un oxo-ester de formule I, 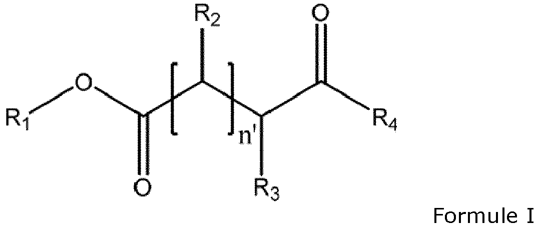 dans laquelle,
   - R₁ est choisi dans le groupe constitué par les alkyles linéaires ou ramifiés comprenant de 1 à 8 atomes de carbone ;
   - R₂, R₃ et R₄ identiques ou différents sont choisis dans le groupe constitué par l'atome d'hydrogène et/ou les alkyles linéaires ou ramifiés comprenant de 1 à 4 atomes de carbone; et
   - n'est un entier naturel de 1 à 4.

Dans un mode de réalisation, le procédé pour dégraisser, nettoyer, protéger, dégripper, faire briller et/ou lubrifier une surface d'un objet selon l'invention comprend au moins une étape d'application sur ladite surface d'une composition comprenant :
a) Au moins un alcane de formule CₙH₂ₙ₊₂ avec 8≤n≤12, et
b) Au moins un oxo-ester de formule I, 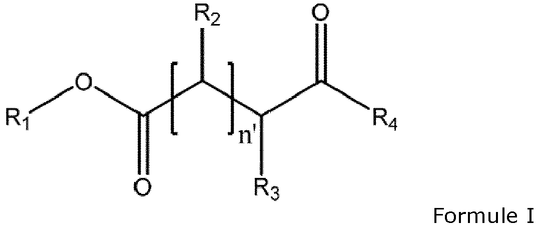 dans laquelle,
   - R₁ est choisi dans le groupe constitué par les alkyles linéaires ou ramifiés comprenant de 1 à 8 atomes de carbone ;
   - R₂, R₃ et R₄ identiques ou différents sont choisis dans le groupe constitué par l'atome d'hydrogène et/ou les alkyles linéaires ou ramifiés comprenant de 1 à 4 atomes de carbone; et
   - n'est un entier naturel de 1 à 4,
   et dans lequel l'alcane de formule CₙH₂ₙ₊₂ avec 8≤n≤12 est linéaire ou ramifié, choisi dans le groupe constitué par le dodécane, le undécane, décane, nonane et/ou un octane.

Dans un mode de réalisation, le procédé pour dégraisser, nettoyer, protéger, dégripper, faire briller et/ou lubrifier une surface d'un objet selon l'invention comprend au moins une étape d'application sur ladite surface d'une composition comprenant :
a) Au moins un alcane de formule CₙH₂ₙ₊₂ avec 8≤n≤12, et
b) Au moins un oxo-ester de formule I, 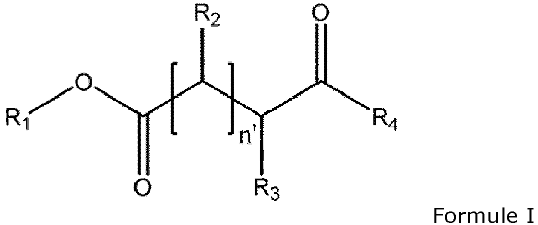 dans laquelle,
   - R₁ est choisi dans le groupe constitué par les alkyles linéaires ou ramifiés comprenant de 1 à 8 atomes de carbone ;
   - R₂, R₃ et R₄ identiques ou différents sont choisis dans le groupe constitué par l'atome d'hydrogène et/ou les alkyles linéaires ou ramifiés comprenant de 1 à 4 atomes de carbone; et
   - n'est un entier naturel de 1 à 4,
et dans lequel l'alcane de formule CₙH₂ₙ₊₂ avec 8≤n≤12 est linéaire ou ramifié, choisi dans le groupe constitué par le dodécane, le undécane, décane, nonane et/ou un octane.

Dans un mode de réalisation, le procédé pour dégraisser, nettoyer, protéger, dégripper, faire briller et/ou lubrifier une surface d'un objet selon l'invention comprend au moins une étape d'application sur ladite surface d'une composition comprenant :
a) Au moins un alcane de formule CₙH₂ₙ₊₂ avec 8≤n≤12, et
b) Au moins un oxo-ester de formule I, 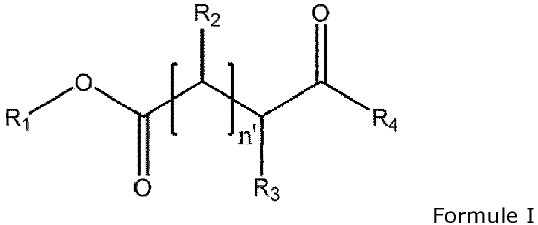 dans laquelle,
   - R₁ est choisi dans le groupe constitué par les alkyles linéaires ou ramifiés comprenant de 1 à 8 atomes de carbone ;
   - R₂, R₃ et R₄ identiques ou différents sont choisis dans le groupe constitué par l'atome d'hydrogène et/ou les alkyles linéaires ou ramifiés comprenant de 1 à 4 atomes de carbone; et
   - n'est un entier naturel de 1 à 4,
et dans lequel l'alcane de formule CₙH₂ₙ₊₂ avec 8≤n≤12 est choisi dans le groupe comprenant : le dodécane, 2-méthyle undécane (CAS 7045-71-8), 3-méthyle undécane (CAS 1002-43-3), 4-méthyle undécane (CAS 2980-69-0), 5-méthyle undécane (CAS 1632-70-8),6-méthyle undécane (CAS 17302-33-9), 2,4-diméthyle décane (CAS 2801-84-5) 4,4-diméthyle décane (CAS 17312-39-9), 3,5-diméthyle décane(17312-48-0), 2,5-diméthyle décane (CAS 17312-50-4), 2,3-diméthyle décane (17312-44-6), 3,3-diméthyle décane (17302-38-4), 3,7-diméthyle décane (CAS 17312-54-8), 3,4,6-triméthyle nonane (CAS 62184-24-1) 3,5,6-triméthyle nonane (CAS 62184-26-3), 3,5,7-triméthyle nonane (CAS 62184-27-4), 2,5,7-triméthyle nonane (CAS 62184-14-9), 2,5,6-triméthyle nonane (CAS 62184-13-8), 2,5,7-triméthyle nonane (CAS 62184-14-9), 2,5,8-triméthyle nonane (CAS 49557-09-7), 3,3,4,5-tétraméthyle octane (CAS 62185-21-1), 2,3,4,5-tétraméthyle octane (CAS 62199-27-3), 2,2,4,5-tétraméthyle octane (CAS 62183-80-6), 2,2,5,7-tétraméthyle octane (CAS 62199-19-3), 2,3,4,7-tétraméthyle octane (CAS 62199-29-5), 2,4,4,7-tétraméthyle octane (CAS 35866-96-7),3-éthyle-4-méthyle nonane (CAS 62184-45-6), 3-éthyle-4,5-diméthyle octane (CAS 62183-72-6), 2,5-diméthyle-6-éthyle octane (CAS 62183-50-0), undécane (CAS 1120-21-4), 2-méthyle décane ( CAS 6975-98-0), le 4-méthyle décane (CAS 2847-72-5), le 3-méthyle décane (13141-34-3), le 5-méthyle décane (CAS 13151-35-4), 2-6 diméthyle nonane (CAS 17302-28-2), 3,7-diméthyle nonane (CAS 17302-32-8), 4,5 diméthyle nonane (CAS 17302-23-7), 2,3-diméthyle nonane (CAS 2884-06-2), 2,4,6-triméthyle octane (CAS 62016-37-9), 2,5,6-triméthyle octane (CAS 62016-14-2), le décane (CAS 124-18-5), le 2-méthyle nonane (CAS 871-83-0), le 4-méthyle nonane (CAS 17301-94-9), le 3-méthyle nonane (CAS 5911-04-6), le 3-éthyle octane (CAS 5881-17-4), le 2,2-diméthyle octane (CAS 15869-87-1), le 2,3 dimethyle octane (CAS 7146-60-3), le 2,5- diméthyle octane (CAS 15869-89-3), le 3,5 diméthyle octane (CAS 15869-93-9), le 4-propyle heptane (CAS 3178-29-8), le 3-éthyle-2-méthyle heptane (CAS 14676-29-0), le 2,2,3-triméthyle heptane (CAS 52896-92-1), le 2,3,5 trimethyle heptane (CAS 20278-85-7), le 2,3,6-trimethyle heptane (CAS 4032-93-3), le 3,3,4-triméthyle heptane (CAS 20278-87-9), le 2,3,4 triméthyle heptane (CAS 52896-95-4), le 2,2,4 triméthyle heptane (CAS : 14720-74-2) le 3,3-diéthyle hexane (CAS 17302-02-2), le 2,2,3,3-tétraméthyle hexane (CAS 13475-81-5), le 3-éthyle-2,2,3-triméthyle pentane (CAS 52897-17-3), nonane (111-84-2), 2-méthyle octane (3221-61-2), 3-méthyle octane (CAS 2216-33-3), 4-méthyle octane (CAS 2216-34-4), 2,4-diméthyle heptane (CAS 2213-23-2), 2,6-diméthyle heptane (CAS 1072-05-5), 2,3-diméthyle heptane (CAS 3074-71-3), 2,5-diméthyle heptane (CAS 2216-30-0), 2,2-diméthyle heptane (CAS 1071-26-7), 2,2,5-triméthyle hexane (CAS 3522-94-9), 2,3,5-triméthyle hexane (CAS 1069-53-0), 2,2,4-triméthyle hexane (CAS 167476-26-5), 2,3,4 triméthyle hexane (CAS 921-47-1), 3-éthyle heptane (CAS 15869-80-4), 4-éthyle heptane (CAS 2216-32-2),octane (CAS 111-65-9), 2,2,4-triméthyle heptane (CAS 592-27-8), 3-méthyle heptane (CAS 589-81-1), 2-méthyle heptane (CAS 592-27-8), 2,3,4-triméthyle pentane (CAS 565-75-3), 2,4 diméthyle hexane (CAS 589-43-5), 2,5 diméthyle hexane (CAS 592-13-2), 3,4 diméthyle hexane (CAS 583-48-2), 3-éthyle hexane (CAS 619-99-8), 4-méthyle heptane (CAS 589-53-7), et leurs mélanges.

Dans un mode de réalisation du procédé selon l'invention, la composition est l'une quelconque des compositions selon l'invention et/ou l'aérosol selon l'invention.

Dans un mode de réalisation, le procédé comprend au moins une étape d'application d'une composition comprenant :
a) au moins un alcane de formule CₙH₂ₙ₊₂ avec n≤12, à une teneur de 40% à 95% (v/v) en volume par rapport au volume total de la composition et
   au moins oxo-ester de formule II : 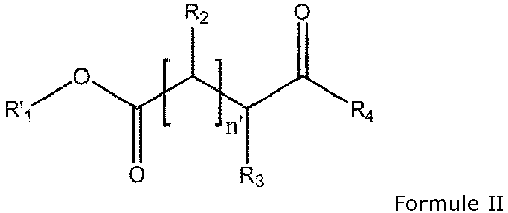 dans laquelle,
   - R'₁ est choisi dans le groupe constitué par les alkyles linéaires ou ramifiés comprenant de 1 à 4 atomes de carbone ;
   - R₂, R₃ et R₄ identiques ou différents sont choisis dans le groupe constitué par l'atome d'hydrogène ou les alkyles linéaires ou ramifiés comprenant de 1 à 4 atomes de carbone; et
   - n'est un entier naturel compris entre 1 et 4,
   la teneur en ledit oxo-ester étant de 5% à 60% (v/v) en volume par rapport au volume total de la composition selon l'invention.

Dans un mode de réalisation, le procédé comprend l'application d'une composition comprenant
a) Au moins un alcane de formule CₙH₂ₙ₊₂ avec n≤ 12, à une teneur de 40% à 95% (v/v) en volume par rapport au volume total de la composition et Au moins un oxo-ester de formule I, 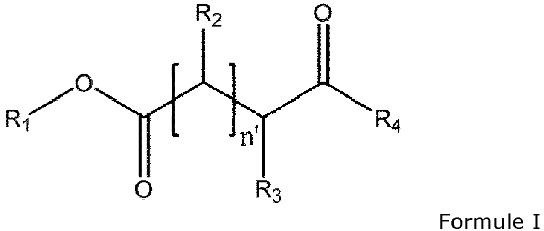 dans laquelle,
   - R₁ est choisi dans le groupe constitué par les alkyles linéaires ou ramifiés comprenant de 1 à 8 atomes de carbone ;
   - R₂, R₃ et R₄ identiques ou différents sont choisis dans le groupe constitué par l'atome d'hydrogène et/ou les alkyles linéaires ou ramifiés comprenant de 1 à 4 atomes de carbone; et
   - n'est un entier naturel de 1 à 4
   la teneur en ledit oxo-ester étant de 5% à 60% (v/v) en volume par rapport au volume total de la composition selon l'invention.
b) Au moins une huile.

Dans un mode de réalisation, le procédé comprend au moins une étape d'application d'une composition comprenant :
a) au moins un alcane de formule CₙH₂ₙ₊₂ avec 8≤n≤12, à une teneur de 40% à 95% (v/v) en volume par rapport au volume total de la composition et
b) au moins oxo-ester de formule II : 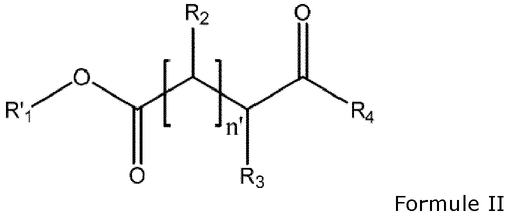 dans laquelle,
   - R'₁ est choisi dans le groupe constitué par les alkyles linéaires ou ramifiés comprenant de 1 à 4 atomes de carbone ;
   - R₂, R₃ et R₄ identiques ou différents sont choisis dans le groupe constitué par l'atome d'hydrogène ou les alkyles linéaires ou ramifiés comprenant de 1 à 4 atomes de carbone; et
   - n'est un entier naturel compris entre 1 et 4,
la teneur en ledit oxo-ester étant de 5% à 60% (v/v) en volume par rapport au volume total de la composition selon l'invention.

Dans un mode de réalisation, le procédé comprend au moins une étape d'application d'une composition comprenant :
c) au moins un alcane de formule CₙH₂ₙ₊₂ avec 8≤n≤12, à une teneur de 40% à 95% (v/v) en volume par rapport au volume total de la composition et
d) au moins oxo-ester de formule II : 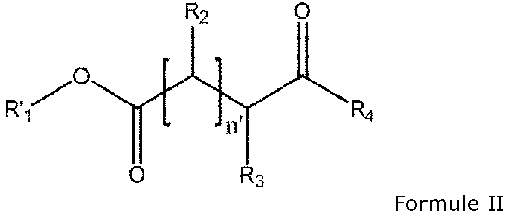 dans laquelle,
   - R'₁ est choisi dans le groupe constitué par les alkyles linéaires ou ramifiés comprenant de 1 à 4 atomes de carbone ;
   - R₂, R₃ et R₄ identiques ou différents sont choisis dans le groupe constitué par l'atome d'hydrogène ou les alkyles linéaires ou ramifiés comprenant de 1 à 4 atomes de carbone; et
   - n'est un entier naturel compris entre 1 et 4,

   la teneur en ledit oxo-ester étant de 5% à 60% (v/v) en volume par rapport au volume total de la composition selon l'invention,
   et dans lequel l'alcane de formule CₙH₂ₙ₊₂ avec 8≤n≤12 est linéaire ou ramifié, choisi dans le groupe constitué par le dodécane, le undécane, décane, nonane et/ou un octane.

Dans un mode de réalisation, le procédé comprend au moins une étape d'application d'une composition comprenant :
a) au moins un alcane de formule CₙH₂ₙ₊₂ avec 8≤n≤12, à une teneur de 40% à 95% (v/v) en volume par rapport au volume total de la composition et
b) au moins oxo-ester de formule II : 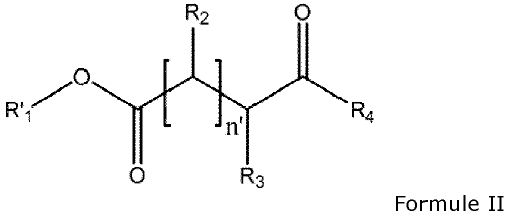 dans laquelle,
   - R'₁ est choisi dans le groupe constitué par les alkyles linéaires ou ramifiés comprenant de 1 à 4 atomes de carbone ;
   - R₂, R₃ et R₄ identiques ou différents sont choisis dans le groupe constitué par l'atome d'hydrogène ou les alkyles linéaires ou ramifiés comprenant de 1 à 4 atomes de carbone; et
   - n'est un entier naturel compris entre 1 et 4,

   la teneur en ledit oxo-ester étant de 5% à 60% (v/v) en volume par rapport au volume total de la composition selon l'invention,
   et dans lequel l'alcane de formule CₙH₂ₙ₊₂ avec 8≤n≤12 est choisi dans le groupe comprenant : le dodécane, 2-méthyle undécane (CAS 7045-71-8), 3-méthyle undécane (CAS 1002-43-3), 4-méthyle undécane (CAS 2980-69-0), 5-méthyle undécane (CAS 1632-70-8),6-méthyle undécane (CAS 17302-33-9), 2,4-diméthyle décane (CAS 2801-84-5) 4,4-diméthyle décane (CAS 17312-39-9), 3,5-diméthyle décane(17312-48-0), 2,5-diméthyle décane (CAS 17312-50-4), 2,3-diméthyle décane (17312-44-6), 3,3-diméthyle décane (17302-38-4), 3,7-diméthyle décane (CAS 17312-54-8), 3,4,6-triméthyle nonane (CAS 62184-24-1) 3,5,6-triméthyle nonane (CAS 62184-26-3), 3,5,7-triméthyle nonane (CAS 62184-27-4), 2,5,7-triméthyle nonane (CAS 62184-14-9), 2,5,6-triméthyle nonane (CAS 62184-13-8), 2,5,7-triméthyle nonane (CAS 62184-14-9), 2,5,8-triméthyle nonane (CAS 49557-09-7), 3,3,4,5-tétraméthyle octane (CAS 62185-21-1), 2,3,4,5-tétraméthyle octane (CAS 62199-27-3), 2,2,4,5-tétraméthyle octane (CAS 62183-80-6), 2,2,5,7-tétraméthyle octane (CAS 62199-19-3), 2,3,4,7-tétraméthyle octane (CAS 62199-29-5), 2,4,4,7-tétraméthyle octane (CAS 35866-96-7),3-éthyle-4-méthyle nonane (CAS 62184-45-6), 3-éthyle-4,5-diméthyle octane (CAS 62183-72-6), 2,5-diméthyle-6-éthyle octane (CAS 62183-50-0), undécane (CAS 1120-21-4), 2-méthyle décane ( CAS 6975-98-0), le 4-méthyle décane (CAS 2847-72-5), le 3-méthyle décane (13141-34-3), le 5-méthyle décane (CAS 13151-35-4), 2-6 diméthyle nonane (CAS 17302-28-2), 3,7-diméthyle nonane (CAS 17302-32-8), 4,5 diméthyle nonane (CAS 17302-23-7), 2,3-diméthyle nonane (CAS 2884-06-2), 2,4,6-triméthyle octane (CAS 62016-37-9), 2,5,6-triméthyle octane (CAS 62016-14-2), le décane (CAS 124-18-5), le 2-méthyle nonane (CAS 871-83-0), le 4-méthyle nonane (CAS 17301-94-9), le 3-méthyle nonane (CAS 5911-04-6), le 3-éthyle octane (CAS 5881-17-4), le 2,2-diméthyle octane (CAS 15869-87-1), le 2,3 dimethyle octane (CAS 7146-60-3), le 2,5- diméthyle octane (CAS 15869-89-3), le 3,5 diméthyle octane (CAS 15869-93-9), le 4-propyle heptane (CAS 3178-29-8), le 3-éthyle-2-méthyle heptane (CAS 14676-29-0), le 2,2,3-triméthyle heptane (CAS 52896-92-1), le 2,3,5 trimethyle heptane (CAS 20278-85-7), le 2,3,6-trimethyle heptane (CAS 4032-93-3), le 3,3,4-triméthyle heptane (CAS 20278-87-9), le 2,3,4 triméthyle heptane (CAS 52896-95-4), le 2,2,4 triméthyle heptane (CAS : 14720-74-2) le 3,3-diéthyle hexane (CAS 17302-02-2), le 2,2,3,3-tétraméthyle hexane (CAS 13475-81-5), le 3-éthyle-2,2,3-triméthyle pentane (CAS 52897-17-3), nonane (111-84-2), 2-méthyle octane (3221-61-2), 3-méthyle octane (CAS 2216-33-3), 4-méthyle octane (CAS 2216-34-4), 2,4-diméthyle heptane (CAS 2213-23-2), 2,6-diméthyle heptane (CAS 1072-05-5), 2,3-diméthyle heptane (CAS 3074-71-3), 2,5-diméthyle heptane (CAS 2216-30-0), 2,2-diméthyle heptane (CAS 1071-26-7), 2,2,5-triméthyle hexane (CAS 3522-94-9), 2,3,5-triméthyle hexane (CAS 1069-53-0), 2,2,4-triméthyle hexane (CAS 167476-26-5), 2,3,4 triméthyle hexane (CAS 921-47-1), 3-éthyle heptane (CAS 15869-80-4), 4-éthyle heptane (CAS 2216-32-2),octane (CAS 111-65-9), 2,2,4-triméthyle heptane (CAS 592-27-8), 3-méthyle heptane (CAS 589-81-1), 2-méthyle heptane (CAS 592-27-8), 2,3,4-triméthyle pentane (CAS 565-75-3), 2,4 diméthyle hexane (CAS 589-43-5), 2,5 diméthyle hexane (CAS 592-13-2), 3,4 diméthyle hexane (CAS 583-48-2), 3-éthyle hexane (CAS 619-99-8), 4-méthyle heptane (CAS 589-53-7), et leurs mélanges.

Dans un mode de réalisation, le procédé comprend l'application d'une composition comprenant
a) Au moins un alcane de formule CₙH₂ₙ₊₂ avec 8≤n≤12, à une teneur de 40% à 95% (v/v) en volume par rapport au volume total de la composition et
b) Au moins un oxo-ester de formule I, 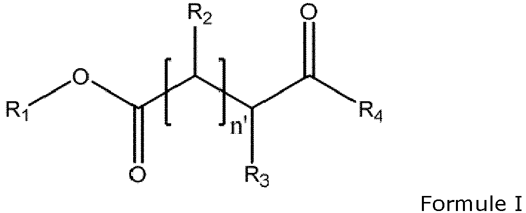 dans laquelle,
   - R₁ est choisi dans le groupe constitué par les alkyles linéaires ou ramifiés comprenant de 1 à 8 atomes de carbone ;
   - R₂, R₃ et R₄ identiques ou différents sont choisis dans le groupe constitué par l'atome d'hydrogène et/ou les alkyles linéaires ou ramifiés comprenant de 1 à 4 atomes de carbone; et
   - n'est un entier naturel de 1 à 4
   la teneur en ledit oxo-ester étant de 5% à 60% (v/v) en volume par rapport au volume total de la composition selon l'invention.
c) Au moins une huile.

Dans un mode de réalisation, le procédé comprend l'application d'une composition comprenant
a) Au moins un alcane de formule CₙH₂ₙ₊₂ avec 8≤n≤12, à une teneur de 40% à 95% (v/v) en volume par rapport au volume total de la composition et
b) Au moins un oxo-ester de formule I, 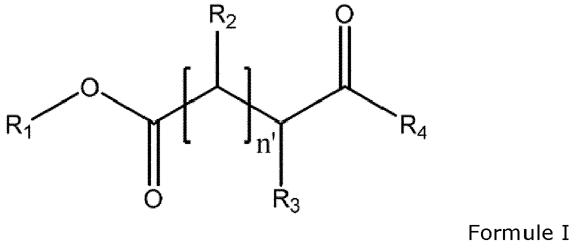 dans laquelle,
   - R₁ est choisi dans le groupe constitué par les alkyles linéaires ou ramifiés comprenant de 1 à 8 atomes de carbone ;
   - R₂, R₃ et R₄ identiques ou différents sont choisis dans le groupe constitué par l'atome d'hydrogène et/ou les alkyles linéaires ou ramifiés comprenant de 1 à 4 atomes de carbone; et
   - n'est un entier naturel de 1 à 4
   la teneur en ledit oxo-ester étant de 5% à 60% (v/v) en volume par rapport au volume total de la composition selon l'invention.
c) Au moins une huile,
   et dans lequel l'alcane de formule CₙH₂ₙ₊₂ avec 8≤n≤12 est linéaire ou ramifié, choisi dans le groupe constitué par le dodécane, le undécane, décane, nonane et/ou un octane.

Dans un mode de réalisation, le procédé comprend l'application d'une composition comprenant
a) Au moins un alcane de formule CₙH₂ₙ₊₂ avec 8≤n≤12, à une teneur de 40% à 95% (v/v) en volume par rapport au volume total de la composition et
b) Au moins un oxo-ester de formule I, 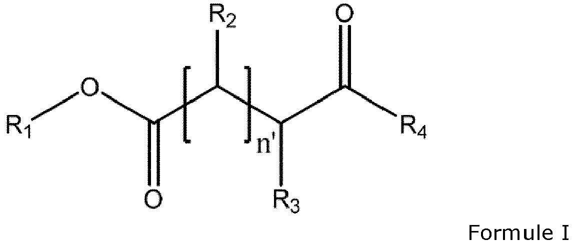 dans laquelle,
   - R₁ est choisi dans le groupe constitué par les alkyles linéaires ou ramifiés comprenant de 1 à 8 atomes de carbone ;
   - R₂, R₃ et R₄ identiques ou différents sont choisis dans le groupe constitué par l'atome d'hydrogène et/ou les alkyles linéaires ou ramifiés comprenant de 1 à 4 atomes de carbone; et
   - n'est un entier naturel de 1 à 4
   la teneur en ledit oxo-ester étant de 5% à 60% (v/v) en volume par rapport au volume total de la composition selon l'invention.
c) Au moins une huile,
et dans lequel l'alcane de formule CₙH₂ₙ₊₂ avec 8≤n≤12 est choisi dans le groupe comprenant : le dodécane, 2-méthyle undécane (CAS 7045-71-8), 3-méthyle undécane (CAS 1002-43-3), 4-méthyle undécane (CAS 2980-69-0), 5-méthyle undécane (CAS 1632-70-8),6-méthyle undécane (CAS 17302-33-9), 2,4-diméthyle décane (CAS 2801-84-5) 4,4-diméthyle décane (CAS 17312-39-9), 3,5-diméthyle décane(17312-48-0), 2,5-diméthyle décane (CAS 17312-50-4), 2,3-diméthyle décane (17312-44-6), 3,3-diméthyle décane (17302-38-4), 3,7-diméthyle décane (CAS 17312-54-8), 3,4,6-triméthyle nonane (CAS 62184-24-1) 3,5,6-triméthyle nonane (CAS 62184-26-3), 3,5,7-triméthyle nonane (CAS 62184-27-4), 2,5,7-triméthyle nonane (CAS 62184-14-9), 2,5,6-triméthyle nonane (CAS 62184-13-8), 2,5,7-triméthyle nonane (CAS 62184-14-9), 2,5,8-triméthyle nonane (CAS 49557-09-7), 3,3,4,5-tétraméthyle octane (CAS 62185-21-1), 2,3,4,5-tétraméthyle octane (CAS 62199-27-3), 2,2,4,5-tétraméthyle octane (CAS 62183-80-6), 2,2,5,7-tétraméthyle octane (CAS 62199-19-3), 2,3,4,7-tétraméthyle octane (CAS 62199-29-5), 2,4,4,7-tétraméthyle octane (CAS 35866-96-7),3-éthyle-4-méthyle nonane (CAS 62184-45-6), 3-éthyle-4,5-diméthyle octane (CAS 62183-72-6), 2,5-diméthyle-6-éthyle octane (CAS 62183-50-0), undécane (CAS 1120-21-4), 2-méthyle décane ( CAS 6975-98-0), le 4-méthyle décane (CAS 2847-72-5), le 3-méthyle décane (13141-34-3), le 5-méthyle décane (CAS 13151-35-4), 2-6 diméthyle nonane (CAS 17302-28-2), 3,7-diméthyle nonane (CAS 17302-32-8), 4,5 diméthyle nonane (CAS 17302-23-7), 2,3-diméthyle nonane (CAS 2884-06-2), 2,4,6-triméthyle octane (CAS 62016-37-9), 2,5,6-triméthyle octane (CAS 62016-14-2), le décane (CAS 124-18-5), le 2-méthyle nonane (CAS 871-83-0), le 4-méthyle nonane (CAS 17301-94-9), le 3-méthyle nonane (CAS 5911-04-6), le 3-éthyle octane (CAS 5881-17-4), le 2,2-diméthyle octane (CAS 15869-87-1), le 2,3 dimethyle octane (CAS 7146-60-3), le 2,5- diméthyle octane (CAS 15869-89-3), le 3,5 diméthyle octane (CAS 15869-93-9), le 4-propyle heptane (CAS 3178-29-8), le 3-éthyle-2-méthyle heptane (CAS 14676-29-0), le 2,2,3-triméthyle heptane (CAS 52896-92-1), le 2,3,5 trimethyle heptane (CAS 20278-85-7), le 2,3,6-trimethyle heptane (CAS 4032-93-3), le 3,3,4-triméthyle heptane (CAS 20278-87-9), le 2,3,4 triméthyle heptane (CAS 52896-95-4), le 2,2,4 triméthyle heptane (CAS : 14720-74-2) le 3,3-diéthyle hexane (CAS 17302-02-2), le 2,2,3,3-tétraméthyle hexane (CAS 13475-81-5), le 3-éthyle-2,2,3-triméthyle pentane (CAS 52897-17-3), nonane (111-84-2), 2-méthyle octane (3221-61-2), 3-méthyle octane (CAS 2216-33-3), 4-méthyle octane (CAS 2216-34-4), 2,4-diméthyle heptane (CAS 2213-23-2), 2,6-diméthyle heptane (CAS 1072-05-5), 2,3-diméthyle heptane (CAS 3074-71-3), 2,5-diméthyle heptane (CAS 2216-30-0), 2,2-diméthyle heptane (CAS 1071-26-7), 2,2,5-triméthyle hexane (CAS 3522-94-9), 2,3,5-triméthyle hexane (CAS 1069-53-0), 2,2,4-triméthyle hexane (CAS 167476-26-5), 2,3,4 triméthyle hexane (CAS 921-47-1), 3-éthyle heptane (CAS 15869-80-4), 4-éthyle heptane (CAS 2216-32-2),octane (CAS 111-65-9), 2,2,4-triméthyle heptane (CAS 592-27-8), 3-méthyle heptane (CAS 589-81-1), 2-méthyle heptane (CAS 592-27-8), 2,3,4-triméthyle pentane (CAS 565-75-3), 2,4 diméthyle hexane (CAS 589-43-5), 2,5 diméthyle hexane (CAS 592-13-2), 3,4 diméthyle hexane (CAS 583-48-2), 3-éthyle hexane (CAS 619-99-8), 4-méthyle heptane (CAS 589-53-7), et leurs mélanges

Dans un mode de réalisation de l'un quelconque des procédés selon l'invention, le au moins un alcane de formule CₙH₂ₙ₊₂ avec 8≤n≤12 est un bioalcane.

Dans un mode de réalisation de l'un quelconque des procédés selon l'invention, les alcanes de formule CnH 2n+2 avec 8≤n≤12 comprennent un mélange de décane et de dodécane.

Dans un mode de réalisation de l'un quelconque des procédés selon l'invention, le au moins un oxo-ester est un lévulinate.

Dans un mode de réalisation de l'un quelconque des procédés selon l'invention, le au moins un oxo-ester est un lévulinate de butyle.et/ou lévulinate d'éthyle.

Dans un mode de réalisation de l'un quelconque des procédés selon l'invention, la composition comprend en outre au moins une huile d'origine végétale.

Dans un mode de réalisation de l'un quelconque des procédés selon l'invention, la teneur en huile est inférieure ou égale à 30% (v/v) en volume d'huile par rapport au volume total de la composition selon l'invention

Dans un mode de réalisation, la surface d'un objet est une surface d'un objet métallique.

Dans un mode de réalisation, l'objet comprend tout ou partie d'un composant électronique, d'une pièce mécanique, d'une pièce automobile, d'un câble, d'une chaine et/ou d'un outil.

Dans un mode de réalisation, le procédé permet de protéger la surface contre l'humidité, la rouille, l'oxydation et/ou le gel.

Dans un mode de réalisation, l'application est réalisée par trempage, par pulvérisation et/ou par pulvérisation aérosol.

Dans un mode de réalisation, l'application a une durée inférieure à 60 minutes.

Dans un mode de réalisation, l'application a une durée inférieure à 15 minutes.

### Description des figures

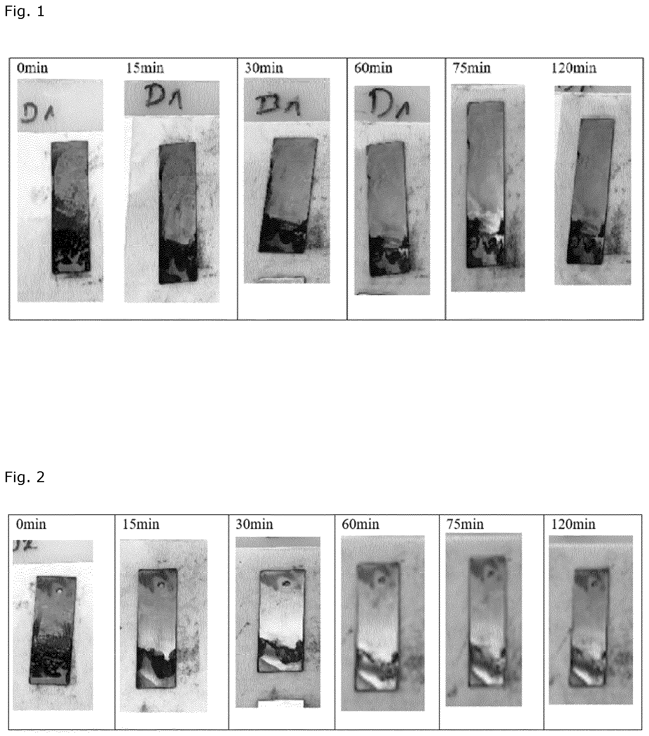

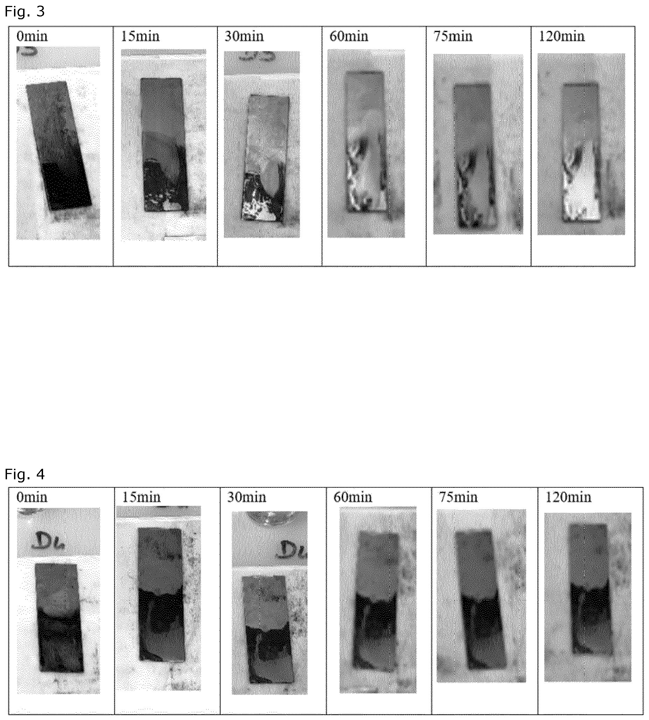

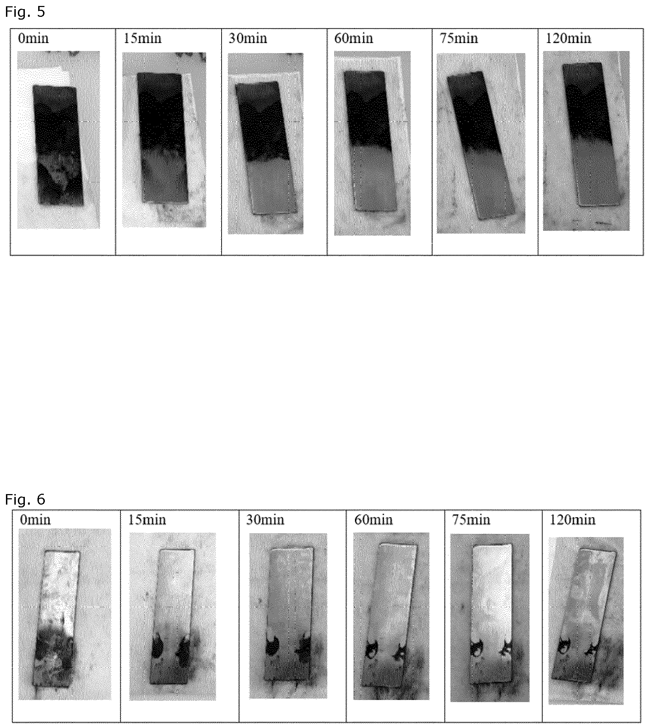

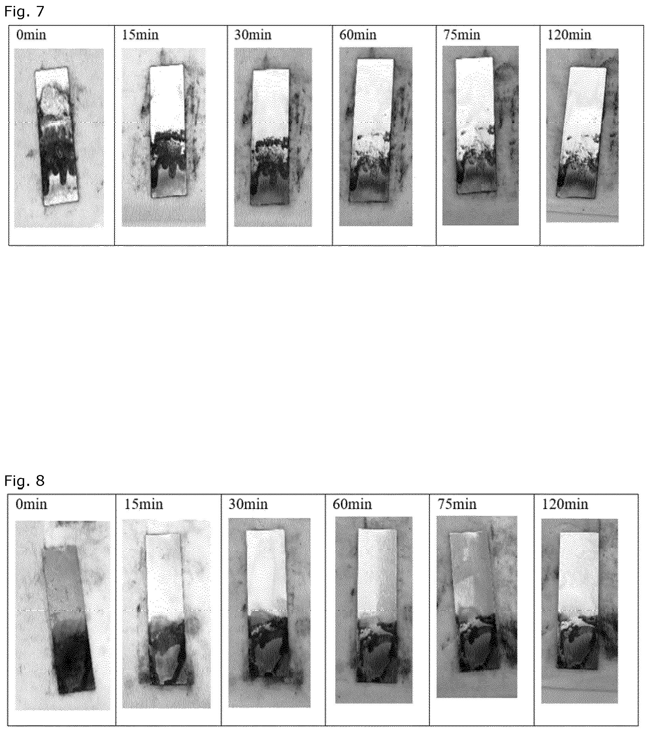

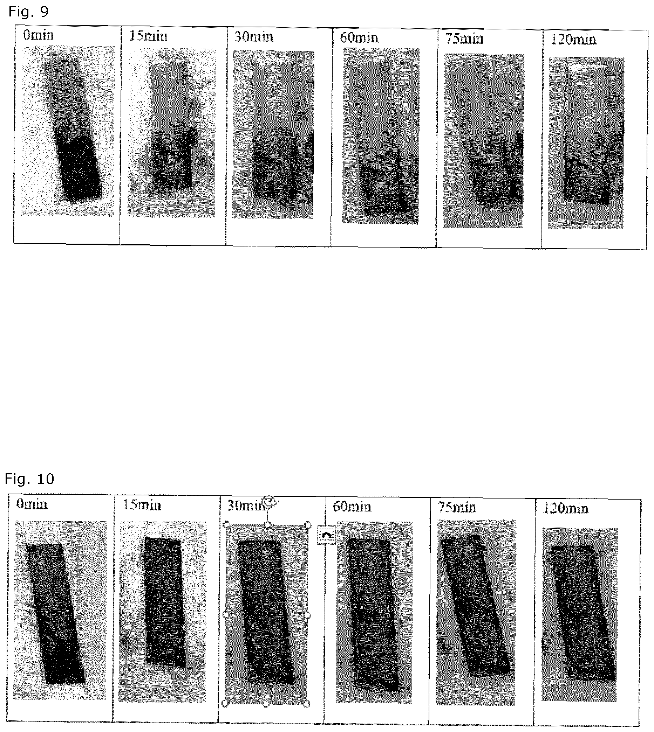

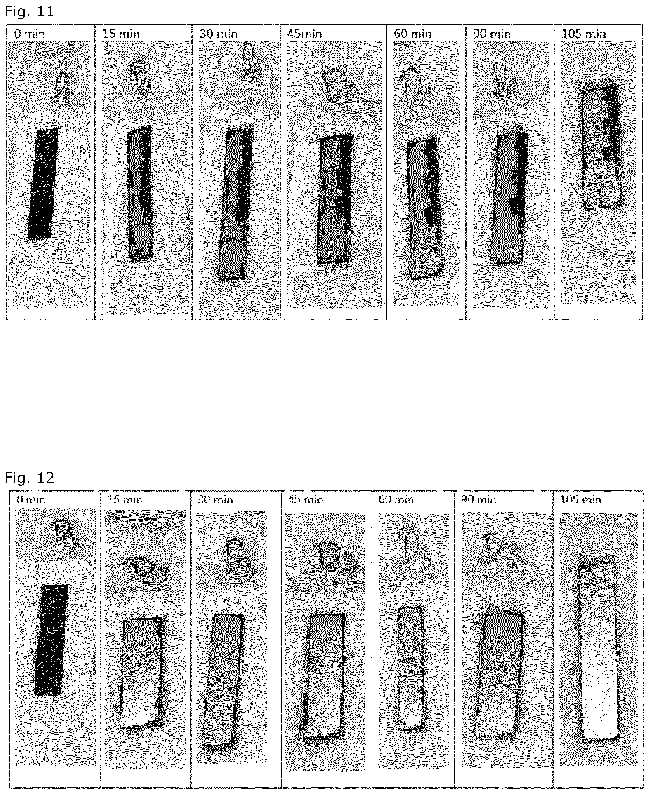

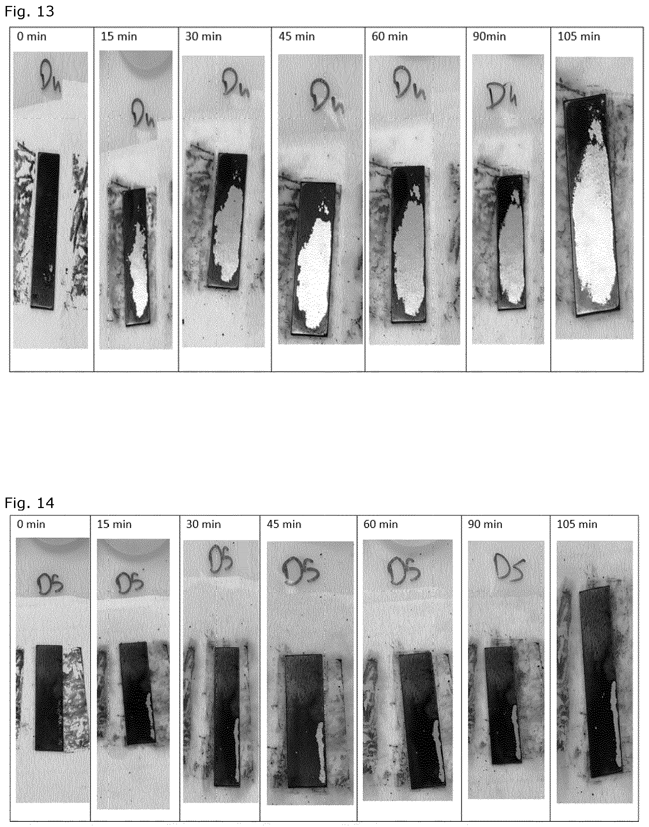

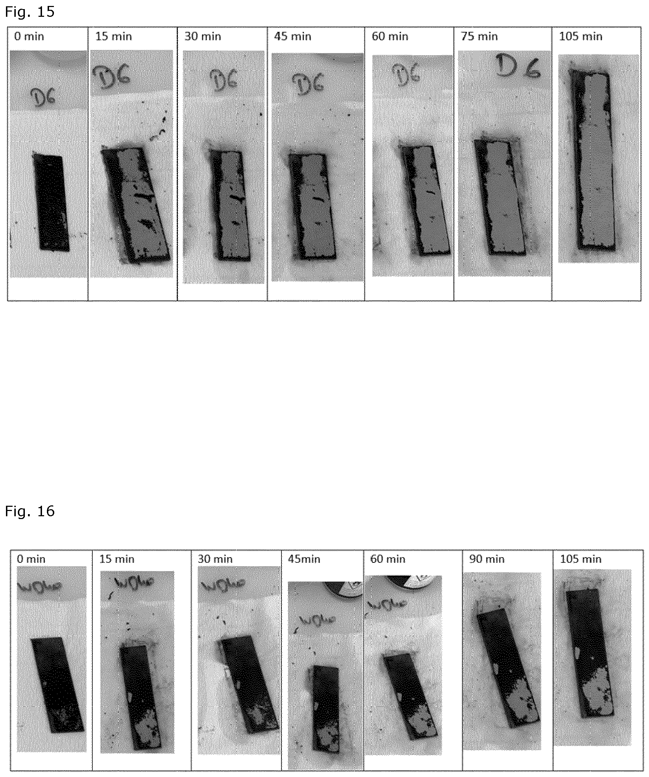

Les figures [Fig. 1] à [Fig. 5] sont des photographies illustrant l'évolution au cours du temps de l'effet dégraissant sur de la graisse brulée, par trempage dans les formules 1 à 4 ou du WD40, respectivement dans cet ordre, selon l'exemple 2.
Les figures [Fig. 6] à [Fig. 10] sont des photographies illustrant l'évolution au cours du temps de l'effet dégraissant sur de la graisse brulée, par pulvérisation des formules 1 à 4 ou du WD40, respectivement dans cet ordre, selon l'exemple 3.
Les figures [Fig. 11] à [Fig. 16] sont des photographies illustrant l'évolution au cours du temps de l'effet dégraissant sur de la graisse cuite, par pulvérisation des formules 1 à 4 ou du WD40, respectivement dans cet ordre, selon l'exemple 4.

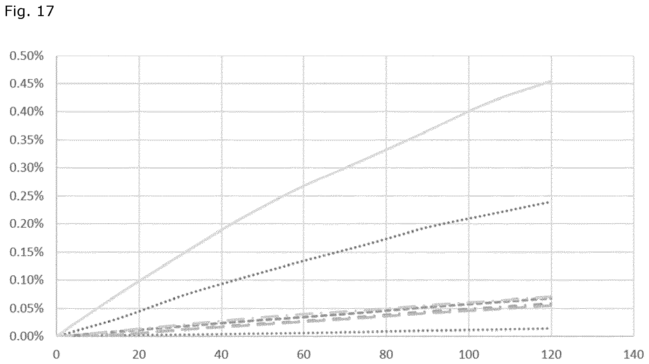

La [Fig. 17] est la courbe d'évolution du pourcentage d'évaporation en fonction du temps selon l'exemple 6, avec, de la courbe ayant la croissance la plus rapide à la plus faible ; la formule 6, le WD40^{®}, la formule 5, la formule 4, la formule 3 et la formule 1.
Dans les exemples, tous les pourcentages sont exprimés en volume, sauf indication contraire, la température est exprimée en degré Celsius sauf indication contraire, et la pression est la pression atmosphérique, sauf indication contraire.

### Exemples

### Exemple 1 : Préparation de compositions selon l'invention.

Les compositions ont été préparées par mélange dans un bécher à température ambiante dans les proportions telles que décrites dans le tableau 1.

Le Vegelight^{®} Silk commercialisé par la société BIOSYNTHIS est un mélange d'alcanes de formule CₙH₂ₙ₊₂ avec n≤ 12 d'origine végétale, comprenant 17% (v/v) en volume de décane et 83% (v/v) en volume de dodécane par rapport au poids total du mélange d'alcane.

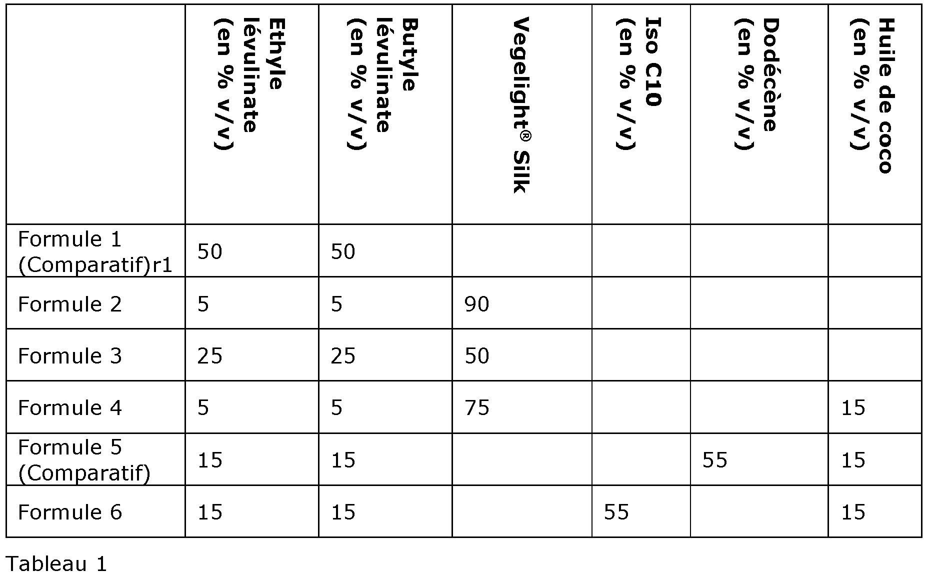

### Exemple 2 : Evaluation de l'effet dégraissant par trempage sur de la graisse brûlée de la composition selon l'invention.

Des plaques en inox ont été trempées dans de la graisse de lithium.

La graisse de lithium déposée a ensuite été brulée à la flamme de chalumeau.

Les plaques ont ensuite été placées dans des éprouvettes contenant l'une des formules selon l'exemple 1 ou du WD40^{®}.

Les plaques ont enfin été photographiées à t=0 min, 15 min, 30 min, 60 min, 75 min, 120 min, comme présentées dans les figures [Fig. 1] à [Fig. 5], dans l'ordre d'apparition dans le tableau ci-dessous respectivement.

Les temps au bout duquel l'effet dégraissant a été observé pour la première fois est repris dans le tableau 2.

**Tableau 2**

| | **Temps (en min)** |
|---|---|
| Formule 1 (comparatif) | Pas d'effet dégraissant |
| Formule 2 | 60 |
| Formule 3 | 30 |
| Formule 4 | 15 |
| WD40^{®} | 15 |

Conclusion : La formule 1, correspondant à l'utilisation d'oxo-ester seul, n'a pas permis d'observer un effet dégraissant par trempage. Avec la formule 2, lorsque la proportion en alcane de formule CₙH₂ₙ₊₂ avec n≤12 est au moins équivalente à la proportion d'oxo-ester, un effet dégraissant est observé au bout de 60 minute. Avec la formule 3, cet effet est observé d'autant plus rapidement lorsque la quantité d'alcane augmente. Avec la formule 4, la présence d'une huile végétale permet d'obtenir un effet dégraissant à un temps comparable à celui de la formulation référence, le WD40^{®}.

### Exemple 3 : Evaluation de l'effet dégraissant sur de la graisse brûlée par pulvérisation de la composition selon l'invention.

Des plaques en inox ont été trempées dans de la graisse de lithium.

La graisse de lithium déposée a ensuite été brulée à la flamme de chalumeau.

Les formules selon l'exemple 1 ou du WD40^{®} ont ensuite été appliquées par pulvérisation en quantités équivalentes sur les plaques.

Les plaques ont enfin été photographiées à t=0 min, 15 min, 30 min, 60 min, 75 min, 120 min, comme présentées dans les figures [Fig. 6] à [Fig. 10] respectivement, dans l'ordre d'apparition dans le tableau ci-dessous.

Les temps au bout duquel l'effet dégraissant a été observé pour la première fois est repris dans le tableau 3.

**Tableau 3**

| | **Temps (en min)** |
|---|---|
| Formule 1 (Comparatif) | 15 |
| Formule 2 | 120 |
| Formule 3 | 120 |
| Formule 4 | 15 |
| WD40^{®} | 15 |

Conclusion : La formule 1 et la formule 4 permettent d'obtenir un effet dégraissant à une durée comparable à celle de la formulation de référence ; le WD40^{®}. Les formules 2 et 3 permettent d'obtenir un effet dégraissant, mais à un temps beaucoup plus long.

### Exemple 4 : Evaluation de l'effet dégraissant sur de la graisse cuite par pulvérisation de la composition selon l'invention.

Des plaques en inox ont été trempées dans de la graisse de lithium.

La graisse de lithium déposée a ensuite été cuites à 300°C.

Les formules selon l'exemple 1 ou du WD40^{®} ont ensuite été appliquées par pulvérisation en quantités équivalentes sur les plaques.

Les plaques ont enfin été photographiées à t=0min, 15min, 30 min, 60 min, 75 min, 120 min, comme présentées dans les figures [Fig. 11] à [Fig. 16] respectivement, dans l'ordre d'apparition dans le tableau ci-dessous.

Les temps au bout duquel l'effet dégraissant a été observé pour la première fois est repris dans le tableau et dans les figures.

**Tableau 4**

| | **Temps (en min)** |
|---|---|
| Formule 1 (Comparatif) | 15 |
| Formule 3 | 15 |
| Formule 4 | 15 |
| Formule 5 (Comparatif) | Pas d'effet dégraissant |
| Formule 6 | 15 |
| WD40^{®} | Pas d'effet dégraissant |

Conclusion : Les formules 3, 4 et 6 selon l'invention ont démontré un effet comparable à celui à la formule 1 comprenant uniquement des oxo-esters. A l'inverse, la formule 5, comprenant un alcène en lieu et place de l'alcane, ainsi que le WD40^{®}, n'ont démontré aucun effet dégraissant.

### Exemple 5 : Mesures de la viscosité et de la densité des compositions selon l'invention.

La densité et les viscosités des formules 1 à 6 et du WD40 ont été mesurées avec un viscosimètre Brookfield (module ULA, vitesse 100 tr/min).

Les résultats sont repris dans le tableau 5.

**Tableau 5**

| | **Densité (20°C g/cm3)** | **Viscosité (centipoises)** | **Viscosité (centistokes)** |
|---|---|---|---|
| Formule 1 (Comparatif) | 0,9926 | 2,41 | 2,42 |
| Formule 2 | 0,7679 | 1,39 | 1,81 |
| Formule 3 | 0,7830 | 1,45 | 1,85 |
| Formule 4 | 0,7879 | 1,99 | 2,52 |
| Formule 5 (Comparatif) | 0,8401 | 2,58 | 2,58 |
| Formule 6 | 0,8231 | 1,67 | 2,02 |
| WD40^{®} | 0,8026 | 2,99 | 3,76 |

### Exemple 6 : Evaluation de la volatilité des compositions selon l'invention.

Le pourcentage d'évaporation en fonction du temps pour les formules selon l'exemple 1 et le WD40^{®} a été mesuré avec un dessiccateur LABOMODERNE THERMOBALANCE KERN DBS 60-3, à 20°C, sous atmosphère contrôlée.

La courbe d'évaporation obtenue est présentée en [Fig. 17].

La formule 6 selon l'invention est la plus volatile. Le WD40^{®} a la seconde meilleure volatilité. La formule comprenant exclusivement des esters de lévulinate est la moins volatile.

Les compositions selon l'invention ont donc une meilleure volatilité que les esters de lévulinate seul.

### Exemple 7 : Evaluation de la compatibilité cutanée d'une composition selon l'invention.

Un test clinique a été réalisé sur un panel de 11 personnes de 23 à 66 ans dans le but de vérifier la compatibilité cutanée du produit par une application d'un patch occlusif (Finn Chamber ^{®} AQUA Patch Test) contenant une quantité de 0,02 ml de la formule 4 selon l'exemple 1 pendant 48h sur la peau du dos du sujet.

Trente minutes après le retrait du patch, une absence totale de manifestations cutanées (érythème, oedème, sécheresse, desquamation, papules, vésicules, effet détergeant ou réflectivité) a été observée par l'opérateur.

La composition selon l'invention est donc compatible avec la peau.

## Revendications

1. Composition **caractérisée en ce qu'**elle comprend :
a) au moins un alcane de formule CₙH₂ₙ₊₂ avec 8≤n≤12, à une teneur de 40% à 95% (v/v) en volume par rapport au volume total de la composition et
b) au moins un oxo-ester de formule I, 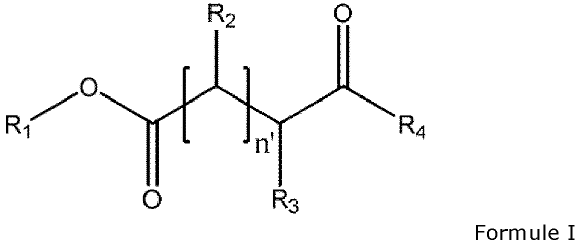 dans laquelle,
- R₁ est choisi dans le groupe constitué par les alkyles linéaires ou ramifiés comprenant de 1 à 8 atomes de carbone ;
- R₂, R₃ et R₄ identiques ou différents sont choisis dans le groupe constitué par l'atome d'hydrogène et/ou les alkyles linéaires ou ramifiés comprenant de 1 à 4 atomes de carbone; et
- n'est un entier naturel de 1 à 4
la teneur en ledit oxo-ester étant de 5% à 60% (v/v) en volume par rapport au volume total de la composition.

2. Composition selon la revendication 1, **caractérisée en ce qu'**au moins un alcane de formule CₙH₂ₙ₊₂ avec 8≤n≤12 est linéaire ou ramifié, choisi dans le groupe constitué par le dodécane, le undécane, décane, nonane et/ou un octane.

3. Composition selon la revendication 1, **caractérisée en ce qu'**au moins un alcane de formule CₙH₂ₙ₊₂ avec 8≤n≤12 est choisi dans le groupe comprenant : le dodécane, 2-méthyle undécane (CAS 7045-71-8), 3-méthyle undécane (CAS 1002-43-3), 4-méthyle undécane (CAS 2980-69-0), 5-méthyle undécane (CAS 1632-70-8),6-méthyle undécane (CAS 17302-33-9), 2,4-diméthyle décane (CAS 2801-84-5) 4,4-diméthyle décane (CAS 17312-39-9), 3,5-diméthyle décane(17312-48-0), 2,5-diméthyle décane (CAS 17312-50-4), 2,3-diméthyle décane (17312-44-6), 3,3-diméthyle décane (17302-38-4), 3,7-diméthyle décane (CAS 17312-54-8), 3,4,6-triméthyle nonane (CAS 62184-24-1) 3,5,6-triméthyle nonane (CAS 62184-26-3), 3,5,7-triméthyle nonane (CAS 62184-27-4), 2,5,7-triméthyle nonane (CAS 62184-14-9), 2,5,6-triméthyle nonane (CAS 62184-13-8), 2,5,7-triméthyle nonane (CAS 62184-14-9), 2,5,8-triméthyle nonane (CAS 49557-09-7), 3,3,4,5-tétraméthyle octane (CAS 62185-21-1), 2,3,4,5-tétraméthyle octane (CAS 62199-27-3), 2,2,4,5-tétraméthyle octane (CAS 62183-80-6), 2,2,5,7-tétraméthyle octane (CAS 62199-19-3), 2,3,4,7-tétraméthyle octane (CAS 62199-29-5), 2,4,4,7-tétraméthyle octane (CAS 35866-96-7),3-éthyle-4-méthyle nonane (CAS 62184-45-6), 3-éthyle-4,5-diméthyle octane (CAS 62183-72-6), 2,5-diméthyle-6-éthyle octane (CAS 62183-50-0), undécane (CAS 1120-21-4), 2-méthyle décane ( CAS 6975-98-0), le 4-méthyle décane (CAS 2847-72-5), le 3-méthyle décane (13141-34-3), le 5-méthyle décane (CAS 13151-35-4), 2-6 diméthyle nonane (CAS 17302-28-2), 3,7-diméthyle nonane (CAS 17302-32-8), 4,5 diméthyle nonane (CAS 17302-23-7), 2,3-diméthyle nonane (CAS 2884-06-2), 2,4,6-triméthyle octane (CAS 62016-37-9), 2,5,6-triméthyle octane (CAS 62016-14-2), le décane (CAS 124-18-5), le 2-méthyle nonane (CAS 871-83-0), le 4-méthyle nonane (CAS 17301-94-9), le 3-méthyle nonane (CAS 5911-04-6), le 3-éthyle octane (CAS 5881-17-4), le 2,2-diméthyle octane (CAS 15869-87-1), le 2,3 dimethyle octane (CAS 7146-60-3), le 2,5- diméthyle octane (CAS 15869-89-3), le 3,5 diméthyle octane (CAS 15869-93-9), le 4-propyle heptane (CAS 3178-29-8), le 3-éthyle-2-méthyle heptane (CAS 14676-29-0), le 2,2,3-triméthyle heptane (CAS 52896-92-1), le 2,3,5 trimethyle heptane (CAS 20278-85-7), le 2,3,6-trimethyle heptane (CAS 4032-93-3), le 3,3,4-triméthyle heptane (CAS 20278-87-9), le 2,3,4 triméthyle heptane (CAS 52896-95-4), le 2,2,4 triméthyle heptane (CAS : 14720-74-2) le 3,3-diéthyle hexane (CAS 17302-02-2), le 2,2,3,3-tétraméthyle hexane (CAS 13475-81-5), le 3-éthyle-2,2,3-triméthyle pentane (CAS 52897-17-3), nonane (111-84-2), 2-méthyle octane (3221-61-2), 3-méthyle octane (CAS 2216-33-3), 4-méthyle octane (CAS 2216-34-4), 2,4-diméthyle heptane (CAS 2213-23-2), 2,6-diméthyle heptane (CAS 1072-05-5), 2,3-diméthyle heptane (CAS 3074-71-3), 2,5-diméthyle heptane (CAS 2216-30-0), 2,2-diméthyle heptane (CAS 1071-26-7), 2,2,5-triméthyle hexane (CAS 3522-94-9), 2,3,5-triméthyle hexane (CAS 1069-53-0), 2,2,4-triméthyle hexane (CAS 167476-26-5), 2,3,4 triméthyle hexane (CAS 921-47-1), 3-éthyle heptane (CAS 15869-80-4), 4-éthyle heptane (CAS 2216-32-2),octane (CAS 111-65-9), 2,2,4-triméthyle heptane (CAS 592-27-8), 3-méthyle heptane (CAS 589-81-1), 2-méthyle heptane (CAS 592-27-8), 2,3,4-triméthyle pentane (CAS 565-75-3), 2,4 diméthyle hexane (CAS 589-43-5), 2,5 diméthyle hexane (CAS 592-13-2), 3,4 diméthyle hexane (CAS 583-48-2), 3-éthyle hexane (CAS 619-99-8), 4-méthyle heptane (CAS 589-53-7), et leurs mélanges.

4. Composition selon l'une quelconque des revendications précédentes, **caractérisée en ce qu'**au moins un alcane de formule CₙH₂ₙ₊₂ avec 8≤n≤12 est un bioalcane.

5. Composition selon l'une quelconque des revendications précédentes, **caractérisée** en ce les alcanes de formule CₙH ₂ₙ₊₂ avec 8≤n≤12 comprennent un mélange de décane et de dodécane.

6. Composition selon l'une quelconque des revendications précédentes, **caractérisée en ce qu'**au moins un oxo-ester est un lévulinate.

7. Composition selon l'une quelconque des revendications 6, **caractérisée en ce que** au moins un oxo-ester est un lévulinate de butyle.et/ou lévulinate d'éthyle.

8. Composition selon l'une quelconque des revendications précédentes, **caractérisée en ce qu'**elle comprend en outre au moins une huile d'origine végétale.

9. Composition selon la revendication 8, **caractérisée** en ce la teneur en huile est inférieure ou égale à 30% (v/v) en volume d'huile par rapport au volume total de la composition selon l'invention.

10. Aerosol comprenant une composition selon l'une quelconque des revendications précédentes et un gaz propulseur.

11. Procédé pour dégraisser, nettoyer, protéger, dégripper, faire briller et/ou lubrifier une surface d'un objet comprenant au moins une étape d'application sur ladite surface d'une composition comprenant :
a) Au moins un alcane de formule CₙH₂ₙ₊₂ avec 8≤n≤12, et
b) Au moins un oxo-ester de formule I, 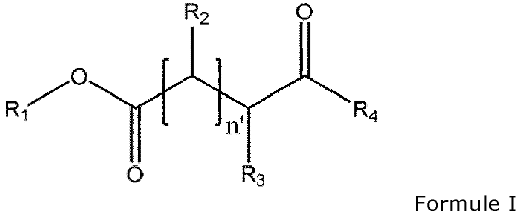 dans laquelle,
- R₁ est choisi dans le groupe constitué par les alkyles linéaires ou ramifiés comprenant de 1 à 8 atomes de carbone ;
- R₂, R₃ et R₄ identiques ou différents sont choisis dans le groupe constitué par l'atome d'hydrogène et/ou les alkyles linéaires ou ramifiés comprenant de 1 à 4 atomes de carbone; et
- n'est un entier naturel de 1 à 4.

12. Procédé selon la revendication 11, dans lequel la composition est une composition telle que définie selon l'une quelconque des revendications 1 à 9 ou un aérosol tel que défini selon la revendication 10.

13. Procédé selon l'une quelconque des revendications 11 ou 12, dans laquelle la surface est une surface d'un objet métallique.
